# EUROPÄISCHE PATENTANMELDUNG

(11) **EP 0 406 163 A1**
(43) Veröffentlichungstag der Anmeldung: **02.01.1991**
(21) Anmeldenummer: 90810445.8
(22) Anmeldetag: 19.06.1990
(51) Int. Cl.: C07D 257/04, A01N 43/713

(54) **Thioxotetrazoline**

(30) Priorität: 26.06.1989 CH 2369/89
(71) Anmelder: CIBA-GEIGY AG, CH-4002 Basel (CH)
(72) Erfinder: Ehrenfreund, Josef, Dr., CH-4123 Allschwil (CH); Stamm, Erich, Dr., CH-4950 Huttwil (CH)

(57) **Zusammenfassung**

Neue 1-Phenyl-5-thioxo-2-tetrazoline der Formel I 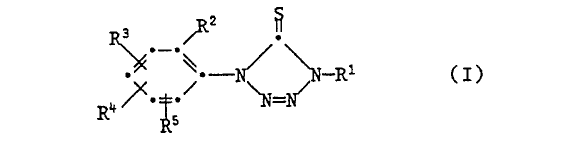 worin
R¹ für C₁-C₈-Alkyl, C₃-C₆-Alkenyl, C₃-C₆-Alkinyl, C₃-C₆-Cycloalkyl, C₅-C₆-Cycloalkenyl, durch C₁-C₄-Alkyl oder Halogen substituiertes C₃-C₆-Cycloalkyl, durch Ci-C₄-Alkyl oder Halogen substituiertes C₅-C₆-Cycloalkenyl, durch Halogen, C₁-C₄-Alkoxy oder Phenyl substituiertes C₃-C₆-Alkenyl, durch Halogen, C₁-C₄-Alkoxy oder Phenyl substituiertes C₃-C₈-Alkinyl oder durch Halogen, C₁-C₄-Alkoxy, C₁-C₄-Alkylthio, C₁-C₄-Alkoxycarbonyl, C₃-C₅-Cycloalkyl, Phenyl, Cyan, Hydroxy, Halogenphenyl, C₁-C₄-Alkylphenyl oder einen heteroaromatischen Rest substituiertes C₁-C₈-Alkyl,
R² für C₁-C₆-Alkyl, C₂-C₆-Alkenyl, C₂-C₆-Alkinyl, C₃-C₆-Cycloalkyl, Cyclopentenyl, Cyclohexenyl oder durch Halogen, C₁-C₄-Alkoxy oder C₁-C₄-Alkylthio substituiertes Ci-C₆-Alkyl,
R³ und R⁴ unabhängig voneinander für Wasserstoff, Halogen, C₁-C₄-Alkyl, C₁-C₄-Halogenalkyl, C₁-C₄-Alkoxy, C₁-C₄-Alkylthio, C₃-C₆-Cycloalkyl, C₂-C₄-Alkenyl, C₂-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH=CH-CH = CH-, -CH₂-CH=CH-, -(CH₂)₄-, -(CH₂)₃-, -O-CH₂-O-, -O-CH₂-CH₂-O-, -CH₂-O-CH₂-, -(CH₂)₂-CH = CH- oder -CH₂-CH=CH-CH₂-Brücke, welche durch ein bis zwei C₁-C₄-Alkylgruppen substituiert sein kann, und R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Alkoxy, C₁-C₄-Halogenalkoxy, C₁-C₄-Halogenalkyl, C₁-C₄-Alkylthio, C₁-C₄-Halogenalkylthio, Di-C₁-C₄-alkyla- mino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(C₁-C₂-Alkyl)-, -N(CHO)-, -CH₂-, -CH(CH₃)- oder -C(CH₃)₂- bedeuten, können als Schädlingsbekämpfungsmittel eingesetzt werden. Vorzugsweise können Insekten und Arachniden bekämpft werden.

## Beschreibung

Die vorliegende Erfindung betrifft neue substituierte 1-Phenyl-5-thioxo-2-tetrazoline, Verfahren und Zwischenprodukte zu ihrer Herstellung, Schädlingsbekämpfungsmittel, welche diese Verbindungen enthalten, sowie ihre Verwendung bei der Bekämpfung von Schädlingen.

Die erfindungsgemässen 1-Phenyl-5-thioxo-2-tetrazoline entsprechen der Formel I 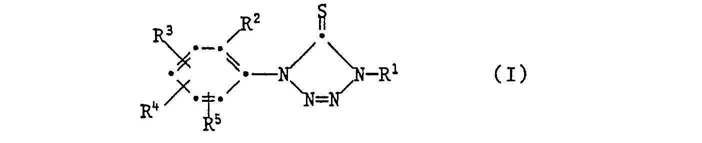 worin
R' für C₁-C₈-Alkyl, Cs-C₆-Alkenyl, Cs-Cs-Alkinyl, C₃-C₆-Cycloalkyl, Cs-C₆-Cycloalkenyl, durch C₁-C₄-Alkyl oder Halogen substituiertes C₃-C₆-Cycloalkyl, durch C₁-C₄-Alkyl oder Halogen substituiertes C₅-C₆-Cycloalkenyl, durch Halogen, C₁-C₄-Alkoxy oder Phenyl substituiertes C₃-C₆-Alkenyl, durch Halogen, C₁-C₄-Alkoxy oder Phenyl substituiertes Cs-Cs-Alkinyl oder durch Halogen, C₁-C₄-Alkoxy, C₁-C₄-Alkylthio, C₁-C₄-Alkoxycarbonyl, C₃-C₆-Cycloalkyl, Phenyl, Cyan, Hydroxy, Halogenphenyl, C₁-C₄-Alkylphenyl oder einen heteroaromatischen Rest substituiertes C₁-C₈-Alkyl,
R² für C₁-C₆-Alkyl, C₂-C₆-Alkenyl, C₂-C₆-Alkinyl, Cs-Cs-Cycloalkyl, Cyclopentenyl, Cyclohexenyl oder durch Halogen, C₁-C₄-Alkoxy oder C₁-C₄-Alkylthio substituiertes Ci-C₆-Alkyl,
R³ und R⁴ unabhängig voneinander für Wasserstoff, Halogen, C₁-C₄-Alkyl, C₁-C₄-Halogenalkyl, C₁C₄-Alkoxy, C₁-C₄-Alkylthio, C₃-C₆-Cycloalkyl, C₂-C₄-Alkenyl, C₂-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH=CH-CH=CH-, -CH₂-CH = CH-, -(CH₂)₄-, -(CH₂)₃-, -O-CH₂-O-, -O-CH₂-CH₂-0-, -CH₂-0-CH₂-, --(CH₂)₂-CH=CH- oder -CH₂-CH=CH-CH₂-Brücke, welche durch ein bis zwei C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Akoxy, Cₗ-C₄-Halogenalkoxy, C₁-C₄-Halogenalkyl, Cₗ-C₄-Alkylthio, C₁-C₄-Halogenalkylthio, Di-Ci-C₄- alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(C₁-C₂-Alkyl)-, -N(CHO)-, -CH₂-, -CH(CH₃)- oder -C-(CH₃)₂- bedeuten.

Aus der Literatur ist die Klasse der 1-Phenyl-5-oxo- oder -5-thioxo-2-tetrazoline als herbizid wirksam oder als Hilfsstoff für photographische Materialien bekannt. Verbindungen des genannten Typs finden sich beispielsweise in der DE-OS 1 447 662, DD-PS 119 322 oder der WO 85/01939.

In der Definition der erfindungsgemässen Formel I sollen die einzelnen generischen Begriffe wie folgt verstanden werden:
Bei den als Substituenten in Betracht kommenden Halogenatomen handelt es sich sowohl um Fluor und Chlor als auch um Brom und Jod, wobei Fluor und Chlor bevorzugt sind. Halogen ist dabei als selbständiger Substituent oder als Teil eines Substituenten zu verstehen wie Halogenalkyl, Halogenalkoxy, Halogenalkylthio oder Halogenphenyl.

Die als Substituenten in Betracht kommenden Alkyl-, Alkylthio- und Alkoxyreste können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl und ihre Isomeren genannt. Als geeignete Alkoxyreste seien unter anderen genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio. Substituenten welche aus mehreren generischen Gruppen zusammengesetzt sind, wie Alkoxyalkyl oder Alkylthioalkyl enthalten entsprechende Kombinationen der genannten Substituentenindividuen.

Die als Substituenten in Betracht kommenden Alkenyle und Alkinyle können geradkettig oder verzweigt sein und eine oder mehrere ungesättigte Bindungen enthalten. Dabei ist es auch möglich, dass zwei- und dreifach ungesättigte Bindungen im gleichen Rest auftreten können. Vorzugsweise enthalten diese ungesättigten Reste zwei bis sechs Kohlenstoffatome. Beispiele solcher Alkenyle und Alkinyle sind unter anderen Vinyl, Allyl, 1-Propenyl, Isopropenyl, Allenyl, Butenyle, Butadienyle, Hexenyle, Hexandienyl, Aethinyl, 1-Propinyl, 2-Propinyl, Butinyle, Pentinyle, Hexinyle, Hexadiinyle, 2-Penten-4-inyl.

Die als Substituenten in Betracht kommenden Phenylalkyle, Phenylalkenyle oder Phenylalkinyle können geradkettig oder verzweigt sein. Geeignete Beispiele sind unter anderen Benzyl, Phenyläthyl, Phenylpropyl, Phenylisopropyl, Phenylbutyl und seine Isomeren, Phenylvinyl, Phenylallyl, Phenylbutenyl, Phenyläthinyl, Phenylpropinyl oder Phenylbutinyl.

Sind die als Substituenten in Betracht kommenden Alkyle, Alkoxyle, Alkylthiole, Alkenyle, Alkinyle oder Phenylalkyle durch Halogen substituiert, so können sie nur teilweise oder auch perhalogeniert sein. Die Nalogensubstitution der Phenylalkyle kann sowohl im Phenylkern als auch in der Alkylkette oder in beiden gleichzeitig erfolgen. Dabei gelten für die Halogene, Alkyle, Alkoxyle, Alkylthiole, Alkenyle, Alkinyle und Phenylalkyle die oben gegebenen Definitionen. Beispiele der Alkylelemente dieser Gruppen sind das ein-bis dreifach durch Fluor, Chlor und/oder Brom substituierte Methyl wie beispielsweise CHF₂ oder CF₃; das ein- bis fünffach durch Fluor, Chlor und/oder Brom substituierte Aethyl wie zum Beispiel CH₂CF₃, CF₂CF₃, CF₂CCI₃, CF₂CHCl₂, CF₂CHF₂, CF₂CFCI₂, CF₂CHBr₂, CF₂CHCIF, CF₂CHBrF oder CCIFCNCIF; das ein-bis siebenfach durch Fluor, Chlor und/oder Brom substituierte Propyl oder Isopropyl wie zum Beispiel CH₂CHBrCN₂Br, CF₂CHFCF₃, CH₂CF₂CF₃ oder CH(CF₃)₂; das ein- bis neunfach durch Fluor, Chlor und/oder Brom substituierte Butyl oder eines seiner Isomeren wie zum Beispiel CF(CF₃)CHFCF₃ oder CH₂-(CF₂)₂CF₃; das ein- bis dreifach durch Fluor, Chlor und/oder Brom substituierte Vinyl, Propinyl oder Pentadiinyl; das ein- bis fünffach durch Fluor, Chlor und/oder Brom substituierte Allyl, 1-Propenyl, Butadienyl oder ein Butinyl; ein ein-bis siebenfach durch Fluor, Chlor und/oder Brom substituiertes Butenyl, Pentadienyl oder Pentinyl; das ein- bis siebenfach durch Fluor, Chlor und/oder Brom substituierte Benzyl; das ein- bis neunfach durch Fluor, Chlor und/oder Brom substituierte Phenyläthyl; das ein- bis elffach durch Fluor, Chlor und/oder Brom substituierte Phenylpropyl oder Phenylisopropyl.

Bei den als Substituenten in Betracht kommenden Cycloalkylen handelt es sich beispielsweise um Cyclopropyl, Cyclobutyl, Cyclopentyl oder Cyclohexyl.

Beispiele für Alkoxycarbonyl- und Alkylcarbonylreste sind Methoxycarbonyl, Aethoxycarbonyl, Propoxycarbonyl, Isopropoxycarbonyl oder Butoxycarbonyl. Alkylcarbonyl steht beispielsweise für Acetyl, Propionyl, Butyryl oder Valeryl, sowie Isomere davon.

Alkylphenyl steht für durch Alkyl substituierte Phenylgruppen. Unter der Bezeichnung heteroaromatische Reste sind in der Definition von R' aromatische Ringe zu verstehen, welche ein oder mehrere Heteroatome als Ringglieder enthalten. Beispiele dafür sind Pyrrol, Pyrazol, Imidazol, Triazole, Tetrazol, Oxazole, Thiazole, Oxadiazole, Thiadiazole, Pyridin, Pyrimidin, Pyrazin, Pyridazin, Triazine oder Chinolin.

Für den Fall, dass wie in der Definition von R' bestimmte Reste ihrerseits weiter substituiert sein können, so tragen sie einen oder mehrere Substituenten, vorzugsweise jedoch nicht mehr als zwei.

Für den Fall, dass R³ und R⁴ gemeinsam eine der angegebenen Brücken bilden, sind R³ und R⁴ an benachbarte Kohlenstoffatome des Phenylringes gebunden.

Unter den Verbindungen der Formel I sind solche Untergruppen herauszuheben, in denen entweder
a) R¹ für C₁-C₅-Alkyl, C₃-C₅-Alkenyl, C₃-C₅-Alkinyl, Cs-Cs-Cycloalkyl oder durch C₁-C₄-Alkoxy, Phenyl oder Cyclopropyl substituiertes C₁-C₄-Alkyl steht, oder
b) R² für C₁-C₄-Alkyl oder Cₛ-C₆-Cycloalkyl steht, oder
c) R³ und R⁴ unabhängig voneinander für Wasserstoff, C₁-C₄-Alkyl, C₂-Cs-Alkinyl, C₂-C₃-Alkenyl oder Cz-C₄-Alkoxyalkyl stehen, oder
d) R⁵ für Wasserstoff, Benzyl, 1-Phenyläthyl, Phenoxy, Halogenphenoxy, Phenylthio, Phenyl, Dichlorpyridyloxy, N-Formylanilinyl oder N-Formylhalogenanilinyl steht.

Von den Verbindungen der Untergruppe c) sind diejenigen herauszuheben, worin einer der Substituenten R³ und R⁴ Wasserstoff bedeutet und der andere die 6-Stellung des Phenylringes besetzt, insbesondere worin R³ für C₁-C₄-Alkyl in 6-Position des Phenylringes steht und R⁴ Wasserstoff bedeutet.

Von den Verbindungen der Untergruppe d) sind diejenigen bevorzugt, worin R⁵ für Wasserstoff, Benzyl, Phenoxy oder Halogenphenoxy in 4-Position des Phenylringes steht.

Als besonders bevorzugte Gruppen von Wirkstoffen der Formel I haben sich solche Verbindungen erwiesen, worin R² für C₁-C₄-Alkyl oder C₅-C₆-Cycloalkyl, R³ für C₁-C₄-Alkyl in 6-Position des Phenylringes, R⁴ für Wasserstoff und R⁵ für Wasserstoff, Benzyl, Phenoxy oder Halogenphenoxy in 4-Position des Phenylringes stehen.

Weiter bevorzugt sind Verbindungen der Formel 1, worin R¹ für C₁-C₃-Alkyl, Cs-Cs-Alkenyl, C₃-C₅-Alkinyl, Cs-C₆-Cycloalkyl oder durch C₁-C₄-Alkoxy, Phenyl oder Cyclopropyl substituiertes C₁-C₄-Alkyl, R² für C₁-C₄-Alkyl oder Cₛ-C₆-Cycloalkyl, R³ und R⁴ unabhängig für Wasserstoff, C₁-C₄-Alkyl, C₂-C₃-Alkinyl, C₂-Cₛ-Alkenyl oder C₂-C₄-Alkoxyalkyl und R⁵ für Wasserstoff, Benzyl, 1-Phenyläthyl, Phenoxy, Halogenphenoxy, Phenylthio, Phenyl, Dichlorpyridyloxy, N-Formylanilinyl oder N-Formylhalogenanilinyl stehen.

Eine ganz besonders ausgezeichnete Gruppe von Wirkstoffen der Formel I ist dadurch gekennzeichnet, dass R¹ für C₁-C₅-Alkyl oder Cₛ-C₆-Cycloalkyl, R² für C₁-C₄-Alkyl oder C₅-C₆-Cycloalkyl, R³ für Cₗ-C₄-Alkyl in 6-Position des Phenylringes, R⁴ für Wasserstoff, R⁵ für Wasserstoff, Benzyl, Phenoxy oder Halogenphenoxy in 4-Position des Phenylringes stehen.

Als bevorzugte Einzelverbindungen der Formel I zu nennen:

1-(2-Aethyl-6-isopropylphenyl)-4-isopropyl-5-isopropyl-5-thioxo-2- tetrazolin, 1-(2,6-Diisopropyl-4-phenoxyphenyl)-4-isopropyl-5-thioxo-2-tetrazolin,
1-(2-Aethyl-6-isopropylphenyl)-4-methyl-5-thioxo-2-tetrazolin,
1-(2,6-Diisopropyl-4-phenoxyphenyl)-4-(2,2-dimethylpropyl)-5-thioxo-2-tetrazolin, 1-(2-Cyclopentyl-6-isopropylphenyl)-4-isopropyl-5-thioxo-2-tetrazolin,
1-(2-Cyclopentyl-6-isopropylphenyl)-4-sec.butyl-5-thioxo-2-tetrazolin,
1-(2,6-Diisopropyl-4-phenoxyphenyl)-4-äthyl-5-thioxo-2-tetrazolin,
1-(2,6-Diisopropylphenyl)-4-(1-methyl-2-methoxyäthyl)-5-thioxo-2-tetrazolin, 1-(2-Methyl-6-isopropylphenyl)-4-isopropyl-5-thioxo-2-tetrazolin,
1-(2-Methyl-6-isopropylphenyl)-4-(2,2-dimethylpropyl)-5-thioxo-2-tetrazolin, 1-(2,6-Diisopropyl-4-phenoxyphenyl)-4-cyclopentyl-5-thioxo-2-tetrazolin,
1-(2,6-Diisopropylphenyl)-4-isopropyl-5-thioxo-2-tetrazolin,
1-(2-Aethyl-6-isopropylphenyl)-4-(2,2-dimethylpropyl)-5-thioxo-2-tetrazolin, 1-(2,6-Diisopropylphenyl)-4-(2,2-dimethylpropyl)-5-thioxo-2-tetrazolin,
1-(2,6-Diisopropylphenyl)-4-(2-cyclohexenyl)-5-thioxo-2-tetrazolin,
1-(2-Methyl-6-isopropylphenyl)-4-sek.butyl-5-thioxo-2-tetrazolin,
1-(2,6-Diisopropyl-4-phenoxyphenyl)-4-cyclopropylmethyl-5-thioxo-2-tetrazolin, 1-(2,6-Diisopropyl-4-benzylphenyl)-4-sek.butyl-5-thioxo-2-tetrazolin,
1-(2,6-Diisopropyl-4-benzylphenyl)-4-(2,2-dimethylpropyl)-5-thioxo-2-tetrazolin, 1-(2,6-Diisopropyl-4-benzylphenyl)-4-isopropyl-5-thioxo-2-tetrazolin,
1-[2,6-Diisopropyl-4-(2-fluorphenoxy)-phenyl]-4-isopropyl-5-thioxo-2-tetrazolin, 1-[2,6-Diisopropyl-4-(1-phenyläthyl)-phenyl]-4-isopropyl-5-thioxo-2-tetrazolin, 1-(2-Cyclopentyl-6-isopropyl-4-phenoxyphenyl)-4-isopropyl-5-thioxo-2-tetrazolin, 1-(2-tert.Butyl-6-methylphenyl)-4-isopropyl-5-thioxo-2-tetrazolin,
1-(2-tert.Butyl-6-methylphenyl)-4-sec.butyl-5-thioxo-2-tetrazolin,
1-(2-tert.Butyl-6-methylphenyl)-4-(2,2-dimethyipropyl)-5-thioxo-2-tetrazolin und 1-(2,6-Diäthylphenyl)-4-isopropyl-5-thioxo-2-tetrazolin.

Die erfindungsgemässen Verbindungen der Formel I können nach im Prinzip bekannten Verfahren hergestellt werden, indem man beispielsweise a) ein 1-Phenyl-5-oxo-2-tetrazolin der Formel II 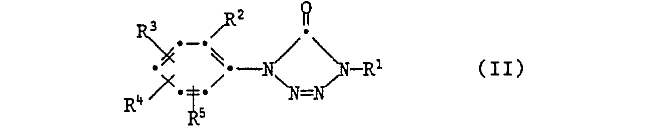 worin R', R², R³, R⁴, und R⁵ die unter Formel I gegebenen Bedeutungen haben, mit einem Thionierungsmittel behandelt, oder

b) ein 1-Phenyl-5-thioxo-2-tetrazolin der Formel III 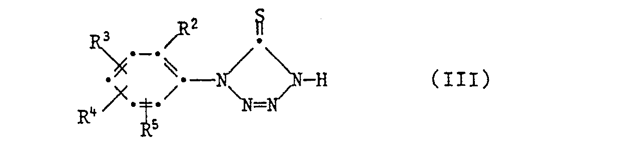 worin R², R3, R⁴ und R⁵ die unter Formel I gegebenen Bedeutungen haben, mit einem Alkylierüngsmittel der Formel IV X - R" (IV) worin R¹¹ die Bedeutung von R¹ wie unter Formel I hat oder für einen in R¹ überführbaren Substituenten steht und X für eine Abgangsgruppe steht, alkyliert und das entstandene 1-Phenyl-5-mercaptotetrazol der Formel V 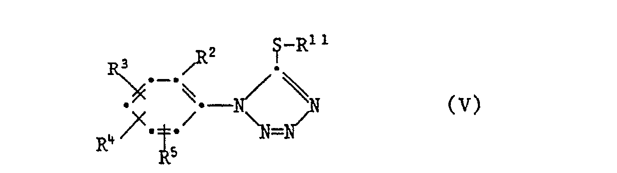 durch eine Umlagerungsreaktion in das entsprechende 1-Phenyl-5-thioxo-2-tetrazolin der Formel la 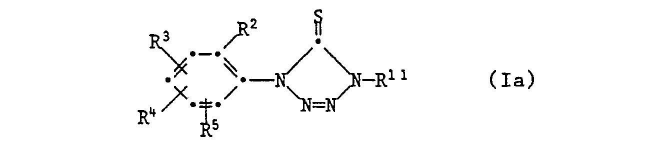 überführt und gewünschtenfalls den Substituenten R" in einen Rest gemäss der Definition von R' umwandelt, oder c) ein 1-Phenyl-5-thioxo-2-tetrazolin der Formel III, in Gegenwart einer Base durch Umsatz mit einem aktivierten, in den Rest R' überführbaren Carbonylvinyl- oder Cyanovinyl-Derivat alkyliert und den eingeführten Substituenten gewünschtenfalls in einen Rest gemäss der Definition von R' umwandelt.

Die Reaktion der Variante a) überführt die Carbonylfunktion des Ausgangsproduktes der Formel II direkt in eine Thiocarbonylfunktion. Analoge Thionierungsreaktionen sind in der Literatur bekannt. Beispielsweise werden Analogieverfahren in Chem. Ber. 118 , 526 (1985) und J. Org. Chem. 41 , 1875 (1976) beschrieben. Das erfindungsgemässe Verfahren a) wird vorzugsweise in Gegenwart eines inerten organischen Lösungsmittels durchgeführt. Geeignete Lösungsmittel sind aromatische Lösungsmittel wie Benzol, Toluol, Xylol, Mesitylen, Chlorbenzol, Dichlorbenzol, Pyridin oder Tetralin; chlorierte Kohlenwasserstoffe wie Methylenchlorid, Chloroform, Tetrachlorkohlenstoff, Aethylenchlorid, Trichloräthan oder Tetrachloräthan; Nitrile wie Acetonitril oder Propionitril oder Aether wie Dioxan, Tetrahydrofuran oder Dimethyläthylenglykol. Die Reaktionstemperaturen liegen im allgemeinen zwischen +20°C und +200°C. Bevorzugt ist ein Bereich zwischen +60°C und +140°C, wobei insbesondere der Siedebereich des Reaktionsgemisches hervorgehoben werden muss. Begünstigt wird die erfindungsgemässe Reaktion auch dadurch, dass man die Thionierungsreaktion unter Einsatz von Ultraschall ausführt und eine Temperatur zwischen + 20 C und dem Siedepunkt des Gemisches wählt. Die Verwendung von Ultraschallquellen zur Begünstigung von Thionierungsreaktionen werden in analogen Verfahren in J. Org. Chem. 46 , 3558 (1981) beschrieben.

Thionierungsmittel sind in der Literatur schon in grosser Anzahl beschrieben worden. In erfindungsgemässen Verfahren können die meisten dieser Mittel eingesetzt werden. Als besonders günstig haben sich als Reagenzien 0,0-Diäthyldithiophosphorsäure (C₂H₅O)₂PS₂H, Borsulfid B₂S₃ oder B₂Ss, Phosphorpentasulfid P₂Ss oder 2,4-Bis-(4-methoxyphenyl)-1,3-dithia-2,4-diphosphetan-2,4-disulfid (Lawesson's Reagenz) erwiesen.

Die Reaktion der Variante b) des erfindungsgemässen Verfahrens kann entweder unter Isolierung des Zwischenproduktes der Formel V in zwei Reaktionsschritten erfolgen oder sie kann direkt in einem einzigen Verfahrensschritt ohne Isolierung des Zwischenprodukts durchgeführt werden. Die Umlagerungsreaktion von V in la kann entweder thermisch durch Erhitzen oder in Gegenwart eines geeigneten Katalysators erfolgen. Als Umlagerungskatalysatoren eignen sich im allgemeinen Komplexverbindungen der Uebergangsmetalle, insbesondere Palladiumkomplexe wie zum Beispiel Bis-benzonitrilo-palladiumchlorid der Formel Pd(CsHs-CN)₂C1₂. Mit Vorteil wird die erfindungsgemässe Verfahrensvariante b) in Gegenwart eines inerten organischen Lösungsmittels bei der Siedetemperatur der Reaktionsmischung durchgeführt. Als Lösungsmittel eignen sich Amide wie Dimethylformamid oder Diäthylformamid, aromatische Lösungsmittel wie Benzol, Toluol, Xylol, Mesitylen, Chlorbenzol, Dichlorbenzol, Pyridin oder Tetralin; chlorierte Kohlenwasserstoffe wie Methylenchlorid, Chloroform, Tetrachlorkohlenstoff, Aethylenchlorid, Trichloräthan oder Tetrachloräthan; Nitrile wie Acetonitril oder Propionitril oder Aether wie Dioxan, Tetrahydrofuran oder Dimethyläthylenglykol.

Die Abgangsgruppen X werden aus der Reihe der für nucleophile Substitutionsreaktionen üblichen Abgangsgruppen ausgewählt. Vorzugweise kommen für X Chlor, Brom, Jod, Toluolsulfonyloxy, Methansulfonyloxy, Trifluormethansulfonyloxy oder Benzolsulfonyloxy in Frage. Unter Substituenten der Definition R", welche in Reste der Definition R' überführt werden können, sind solche Radikale zu verstehen, die durch einfache Reaktionen wie zum Beispiel Hydrolyse, Hydrierung oder Additionen entsprechend abgewandelt werden können. Beispiele dafür sind Carbonylgruppen-haltige Radikale, welche nach der Substitution halogeniert, hydriert oder hydrolysiert werden, oder ungesättigte Substituenten welche beispielsweise durch 1,2-Addition in gesättigte Derivate überführt werden, wie durch Halogen-, Halogenwasserstoff- oder Alkoholaddition.

In der Verfahrensvariante c) werden durch 1,4-Addition solche Radikale R' mit dem Zwischenprodukt der Formel III verknüpft, die mindestens 3-Kohlenstoffatome enthalten und unter den Bedingungen einer Michaeladdition gebildet werden können. Als aktivierte Michaelreagenzien müssen in den Alkylierungsmittein die Strukturelemente Carbonylvinyl 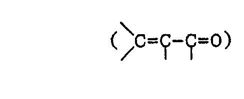 oder Cyanovinyl 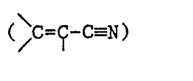 vorhanden sein. Die entstehenden Produkte können gewünschtenfalls durch übliche Derivatisierungsreaktionen in andere Reste R' umgewandelt werden. Zur Durchführung der Variante c) geeignete Basen sind vorzugsweise tert.Amine wie Triäthylamin, Pyridin oder Dimethylaminopyridin, aber auch Alkalialkoholate oder Alkalihydride. Mit Vorteil wird das Verfahren c in einem inerten Lösungsmittel bei der Siedetemperatur der Reaktionsmischung durchgeführt. Solche Lösungsmittel sind Alkohole wie Methanol, Aethanol, Propanol oder Isopropanol, Amide wie Dimethylformamid oder Diäthylformamid, aromatische Lösungsmittel wie Benzol, Toluol, Xylol, Mesitylen, Chlorbenzol, Dichlorbenzol, Pyridin oder Tetralin; chlorierte Kohlenwasserstoffe wie Methylenchlorid, Chloroform, Tetrachlorkohlenstoff, Aethylenchlorid, Trichloräthan oder Tetrachloräthan; Nitrile wie Acetonitril oder Propionitril oder Aether wie Dioxan, Tetrahydrofuran oder Dimethyläthylenglykol.

Grundsätzlich steht bei der Herstellung der Verbindungen der Formel I auch die Möglichkeit offen, die Substituenten R¹, R², R³, R⁴ und R⁵ durch geeignete Derivatisierungsreaktionen oder Substitutionsreaktionen in andere Reste unter der entsprechenden Definition zu überführen. Beispielsweise kann so ein unter R³ oder R⁴ fallender Halogensubstituent durch eine nucleophile Substitutionsreaktion durch einen Rest -Z-R⁶ ersetzt werden.

Die Ausgangsmaterialien der Formel IV sind bekannt und im Handel erhältlich oder sie können nach an sich bekannten Verfahren erhalten werden.

Die Ausgangsverbindungen der Formel II sind nach dem folgenden Schema 1 aus bekannten Anilinen der Formel VI über Isocyanate der Formel VII und 1-Phenyl-5-oxotetrazoline der Formel VIII erhältlich. 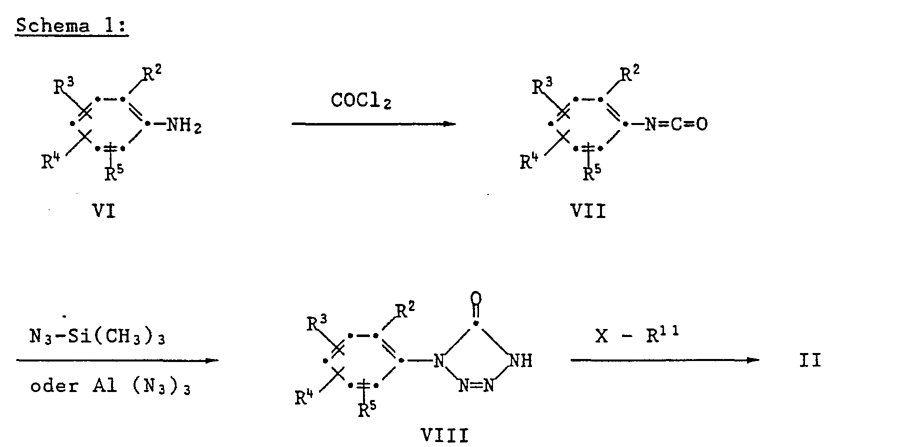 R², R³, R⁴ und R⁵ haben die unter Formel I, X und R" die unter Formel IV,gegebenen Bedeutungen.

_{.} Die Ausgangsverbindungen der Formel III können nach Schema 2 aus bekannten Anilinen der Formel VI über Thioisocyanate der Formel IX oder Anilinocarbodithioate der Formel X erhalten werden. 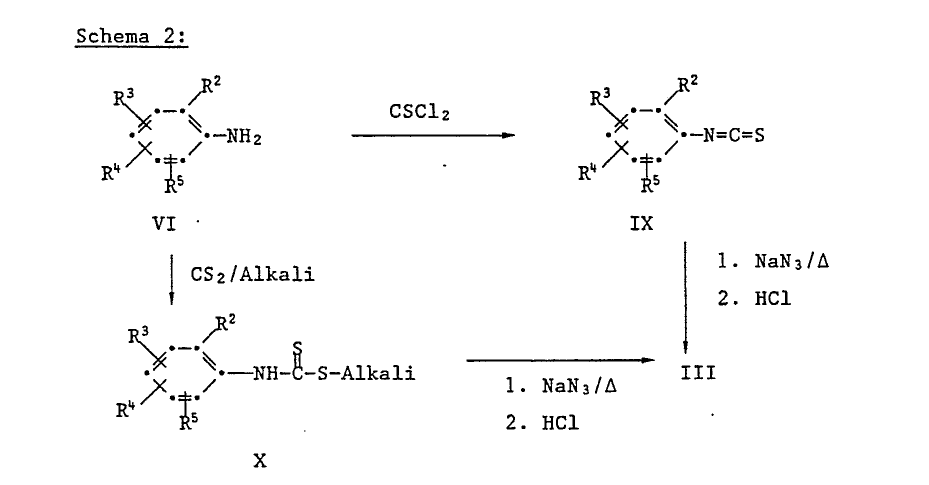 R2, R³, R⁴ and R⁵ haben die unter Formel gegebenen Bedeutungen.

Die Zwischenproduckt der Formel 111 können aus derjenigen der Formel II ebenfalls durch Behandlung mit Thionierungsmitteln erhalten werden, wie sie für die Variante a) des erfindungsgemässen Herstellungverfahren verwendet werden.

Die Zwischenproduckte der Formeln 11 und VIII sind neu. Sie wurden speziell für die Synthese der Virbindungen der Formel I entwickelt. Sie bilden daher einen Gegenstand der vorliegenden Erfindung.

Es wurde nun gefunden, dass die erfindungsgemässen Verbindungen der Formel 1 bei günstiger Warmblüter- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus-und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schäldlinge oder erst nach einiger Zeit, beipielsweise bei einer Häutung, oder in einer verminderten Eiablage und/oder Schlupfrate zeigen. Zu den oben erwähnten Schädlingen gehören:
aus der Ordnung Lepidoptera zum Beispiel Amylois spp., Coleophora spp., Yponomeuta spp., Prays spp., Lyonetia spp., Keiferia lycopersicella, Pectinophora gossypiella, Plutella xylostella, Leucoptera scitella, Lithocollethis spp., Aegeria spp., Synanthedon spp., Adoxophyes spp., Pieris spp., Archips spp., Argyrotaenia spp., Choristoneura spp., Pandemis spp., Sparganothis spp., Cnephasia spp., Acleris spp., Tortrix spp., Cochylis spp., Eupoecilia ambiguella, Hedya nubiferana, Lobesia botrana, Eucosma spp., Cydia spp., Grapholita spp., Pammene spp., Malacosoma spp., Manduca sexta, Chilo spp., Diatraea spp., Crocidolomia binotalis, Ostrinia nubilalis, Cadra cautella, Ephestia spp., Operophtera spp., Thaumetopoea spp., Euproctis spp., Lymantria spp., Agrotis spp., Euxoa spp., Mamestra brassicae, Panolis flammea, Busseola fusca, Sesamia spp., Spodoptera spp., Heliothis spp., Earias spp., Autographa spp., Trichoplusia ni, Cryptophlebia leucotreta, Phthorimaea operculella, Diparopsis castanea, Alabama argillaceae, Anticarsia gemmatalis und Hellula undalis, Cnaphalocrocis spp., Scirpophaga spp., Hyphantria cunea, Carposina nipponensis, Phtorimaea operculella, Cryptophlebia leucotreta, Clysia ambiguella und Pieris rapae; aus der Ordnung Coleoptera zum Beispiel Sitotroga spp., Leptinotarsa decemlineata, Diabrotica spp., Agriotes spp., Anthonomus spp., Cosmopolites spp., Dermestes spp., Epilachna spp., Orycaephilus spp., Sitophilus spp., Otierhynchus spp., Tribolium spp., Tenebrio spp., Melolontha spp., Popillia spp., Rhizopertha spp., Trogoderma spp., Curculio spp., Eremnus spp. und Phlyctinus spp., Lissorhoptrus spp., Chaetocnema tibialis, Psylliodes spp., Atomaria linearis und Scarabeidae; aus der Ordnung Orthoptera zum Beispiel Blatta spp., Periplaneta spp., Leucophaea maderae, Blattella spp., Gryllotalpa spp., Locusta spp. und Schistocerca spp.; aus der Ordnung Isoptera zum Beispiel Reticulitermes spp.; aus der Ordnung Psocoptera zum Beispiel Liposcelis spp.; aus der Ordnung Anoplura zum Beispiel Phylloxera spp., Pemphigus spp., Pediculus spp., Haematopinus spp. und Linognathus spp.; aus der Ordnung Mallophaga zum Beispiel Trichodectes spp. und Damalinea spp.; aus der Ordnung Thysanoptera zum Beispiel Hercinothrips spp., Thrips tabaci, Taeniothrips spp. und Scirtothrips aurantii, Frank Liniella spp. und Thrips palmi; aus der Ordnung Heteroptera zum Beispiel Eurygaster 'spp., Dysdercus spp., Piesma spp., Cimex spp., Rhodnius spp., Triatoma spp., Nezzara spp., Scotinophara spp., Leptocorisa spp., Euchistus spp., Sahlbergella singularis und Distantiella theobroma; aus der Ordnung Homoptera zum Beispiel Aleurodes brassicae, Bemisia tabaci, Trialeurodes vaporariorum, Aphididae, Empoasca spp., Nephotettix spp., Laodelphax spp., Nilaparvata spp., Aonidiella spp., Lecanium corni, Saissetia spp., Aspidiotus spp., Pseudococcus spp., Planococcus spp., Pseudaulacaspis spp., Quadraspidiotus spp., Psylla spp., Chrysomphalus aonidium, Aleurothrixus floccosus, Trioza erytreae, Eriosoma larigerum, Unaspis citri, Ceroplaster spp. und Partatoria spp., Lepidosaphes spp., Erythroneura spp., Gascardia spp., Coccus hesperidum, Pulvinaria aethiopica, Schizaphis spp., Aphis spp., Sitobion spp., Macrosiphus spp., Rhopalosiphum spp., Myzus spp., Pemphigus spp., Scaphoideus spp. und Chrysophalus dictyospermi; aus der Ordnung Hymenoptera zum Beispiel Diprion spp., Hoplocampa spp., Lasius spp., Monomorium pharaonis, Vespa spp., Neodiprion spp. und Solenopsis spp., Atta spp., Acromyrex, Diprionidae, Gilpinia polytoma und Cephus spp., aus der Ordnung Diptera zum Beispiel Aedes spp., Culex spp., Drosophila melanogaster, Musca spp., Fannia spp., Calliphora erythrocephala, Lucilia spp., Chrysomyia spp., Cuterebra spp., Glossina spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis spp., Dacus spp., Tipula spp., Liriomyza spp., Melanagromyza spp., Antherigona soccata, Sciara spp., Rhagoletis pomonella und Orseolina spp.; aus der Ordnung Siphonaptera zum Beispiel Xenopsylla cheopis und Ceratophyllus spp.; aus der Ordnung der Acarina zum Beispiel Panonychus spp., Tetranychus spp., Tarsonemus spp., Bryobia praetiosa, Acarus siro, Argas spp., Ornithodoros spp., Dermanyssus gallinae, Eriophyes spp., Phyllocoptruta oleivora, Boophilus spp., Rhipicephalus spp., Amblyomma spp., Hyalomma spp., Ixodes spp., Psoroptes spp., Chorioptes spp., Saracoptes spp., Aceria sheldoni, Polyphagotarsonemus latus, Eotetranychus carpini und Brevipalpus spp., Calipitrimerus spp., Aculus schlechtendali, Rhizoglyphus spp. und Olygonychus pratensis und aus der Ordnung der Thysanura zum Beispiel Lepisma saccharina.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu Emulsionskonzentraten, direkt versprüh- oder verdünnbaren Lösungen, verdünnten Emulsionen, Spritzpulvern' löslichen Pulvern, Stäubemitteln, Granulaten, auch Verkapselungen in polymeren Stoffen in bekannter Weise verarbeitet werden. Die Anwendungsverfahren' wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen Cₛ bis C₁₂, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren (C_{1O}-C₂₂), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium-oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlen wasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propyleriglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten..

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"1985 International Mc Cutcheon's Emulsifiers & Detergents", Glen Rock NJ USA, 1985",
H. Stache, "Tensid-Taschenbuch", 2. Aufl., C. Hanser Verlag München, Wien 1981,
M. and J. Ash. "Encyclopedia of Surfactants", Vol. 1-111, Chemical Publishing Co., New York, 1980-1981.

Die pestiziden Zubereitungen enthalten in der Regel 0,1 bis 99 % insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)

### Emulgierbare Konzentrate

Aktiver Wirkstoff: 1 bis 20 %, bevorzugt 5 bis 10 % oberflächenaktives Mittel: 5 bis 30 %, vorzugsweise 10 bis 20 % flüssiges Trägermittel: 50 bis 94 %, vorzugsweise 70 bis 85 % Stäube: Aktiver Wirkstoff: 0,1 bis 10 %, vorzugsweise 0,1 bis 1 % festes Trägermittel: 99,9 bis 90 %, vorzugsweise 99,9 bis 99 % Suspension-Konzentrate Aktiver Wirkstoff: 5 bis 75 %, vorzugsweise 10 bis 50 % Wasser: 94 bis 24 %, vorzugsweise 88 bis 30 % oberflächenaktives Mittel: 1 bis 40 %, vorzugsweise 2 bis 30 % Benetzbare Pulver Aktiver Wirkstoff: 0,5 bis 90 %, vorzugsweise 1 bis 80 % oberflächenaktives Mittel: 0,5 bis 20 %, vorzugsweise 1 bis 15 % festes Trägermaterial: 5 bis 95 %, vorzugsweise 15 bis 90 % Granulate aktiver Wirkstoff: 0,5 bis 30 %, vorzugsweise 3 bis 15 % festes Trägermittel: 99,5 bis 70 %, vorzugsweise 97 bis 85 %

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

### Herstellungsbeispiele

### Beispiel H1: 1-(2,6-Diisopropylphenyl)-4-isopropyl-5-thioxo-2-tetrazolin

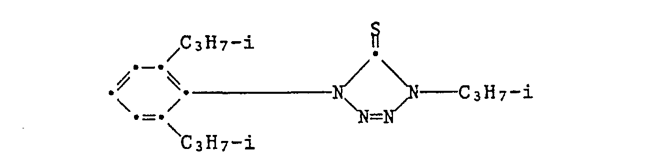

Eine Mischung von 5,6 g 1-(2,6-Diisopropylphenyl)-4-isopropyl-5-oxo-2-tetrazolin und 5,1 g 2,4-Bis-(4-methoxyphenyl)-1,3-dithia-2,4-di-phosphetan-2,4-disulfid (Lawesson's Reagenz) werden in 60 ml trockenem Toluol für 24 Stunden zum Rückfluss erhitzt. Nach Abdampfen des Lösungsmittels wird der Rückstand durch Chromatographie an 500 g Kieselgel mit Hexan/Aethylacetat (19:1) als Elutionsmittel gereinigt. Man erhält nach Kristallisation aus Hexan 4,2 g 1-(2,6-Diisopropylphenyl)-4-isopropyl-5-thioxo-2-tetrazolin als farblose Kristalle, Smp. 109-111 C.

### Beispiel H2: 1-(2,6-Diisopropylphenyl)-4-isopropyl-5-oxo-2-tetrazolin

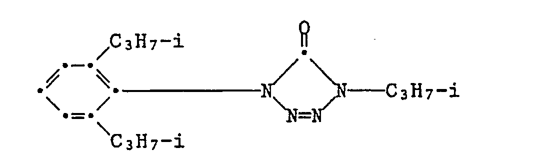

a) 1-(2,6-Diisopropylphenyl)-5-oxo-2-tetrazolin 37,1 g 2,6-Diisopropylphenylisocyanat und 50 ml Trimethylsilylazid werden 24 Stunden bei +140°C gerührt. Anschliessend wird auf Raumtemperatur gekühlt und jeweils mit 250 ml Toluol und 250 ml Wasser verrührt. Nach 1 Stunde werden die Phasen getrennt und die Wasserphase mit Toluol extrahiert. Die vereinigten organischen Phasen werden viermal mit je 250 ml 15 %-Natriumhydroxidlösung extrahiert, anschliessend werden die wässrigen Phasen vereinigt und mit Aether gewaschen. Anschliessend wird mit halbkonzentrierter Salzsäure angesäuert, wobei das rohe 1-(2,6-Diisopropylphenyl)-5-oxo-2-tetrazolin ausfällt. Dieses wird aus einer Mischung von Hexan und Toluol im Verhältnis 10:1 umkristallisiert. Schmelzpunkt 177-178,5°C.
b) 14,3 g 1-(2,6-Diisopropylphenyl)-5-oxo-2-tetrazolin, 12,8 g Isopropyljodid, 10,4 g pulverisiertes Kaliumcarbonat werden in 140 ml Dimethylformamid gelöst und 2 Stunden bei + 60 C gerührt. Man giesst die erhaltene Suspension auf Wasser und extrahiert dreimal mit Aether. Die vereinigten Aetherphasen werden mit Wasser und gesättigter Kochsalzlösung gewaschen, über Na₂S0₄ getrocknet und eingedampft. Zur Entfernung des als Nebenprodukt gebildeten 1-(2,6-Diisopropylphenyl)-5-ispropyloxy-tetra- zols wird an Kieselgel mit dem Laufmittel CH₂C1₂/Cyclohexan (3:1) chromatographiert. Man erhält so 8,7 g 1-(2,6-Diisopropylphenyl)-4-isopropyl-5-oxo-2-tetrazolin vom Schmelzpunkt 60-62°C.

Auf analoge Weise werden die in den folgenden Tabellen 1 und 2 aufgelisteten Zwischenprodukte der Formeln II und VIII und Wirkstoffe der Formel I erhalten.

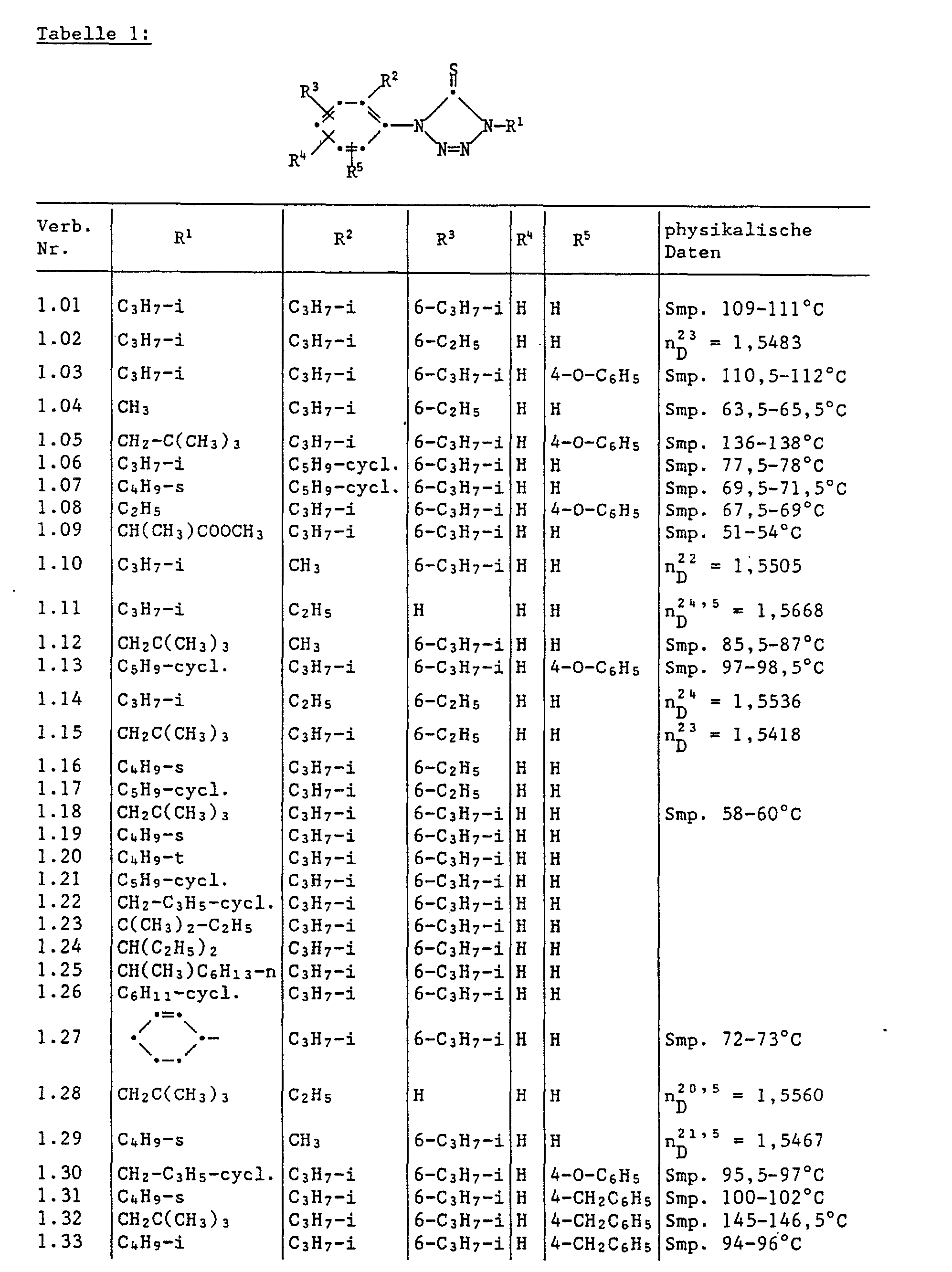

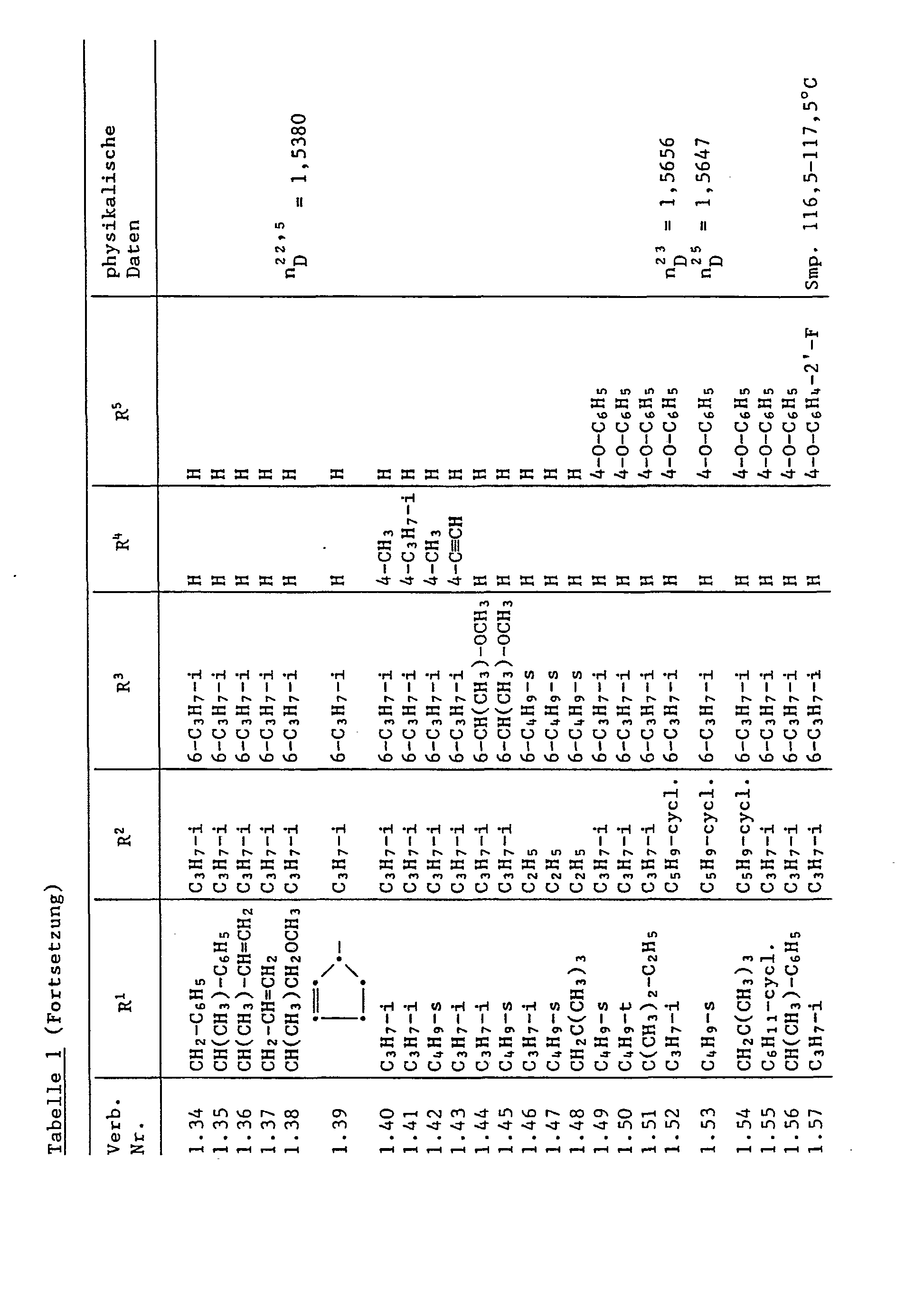

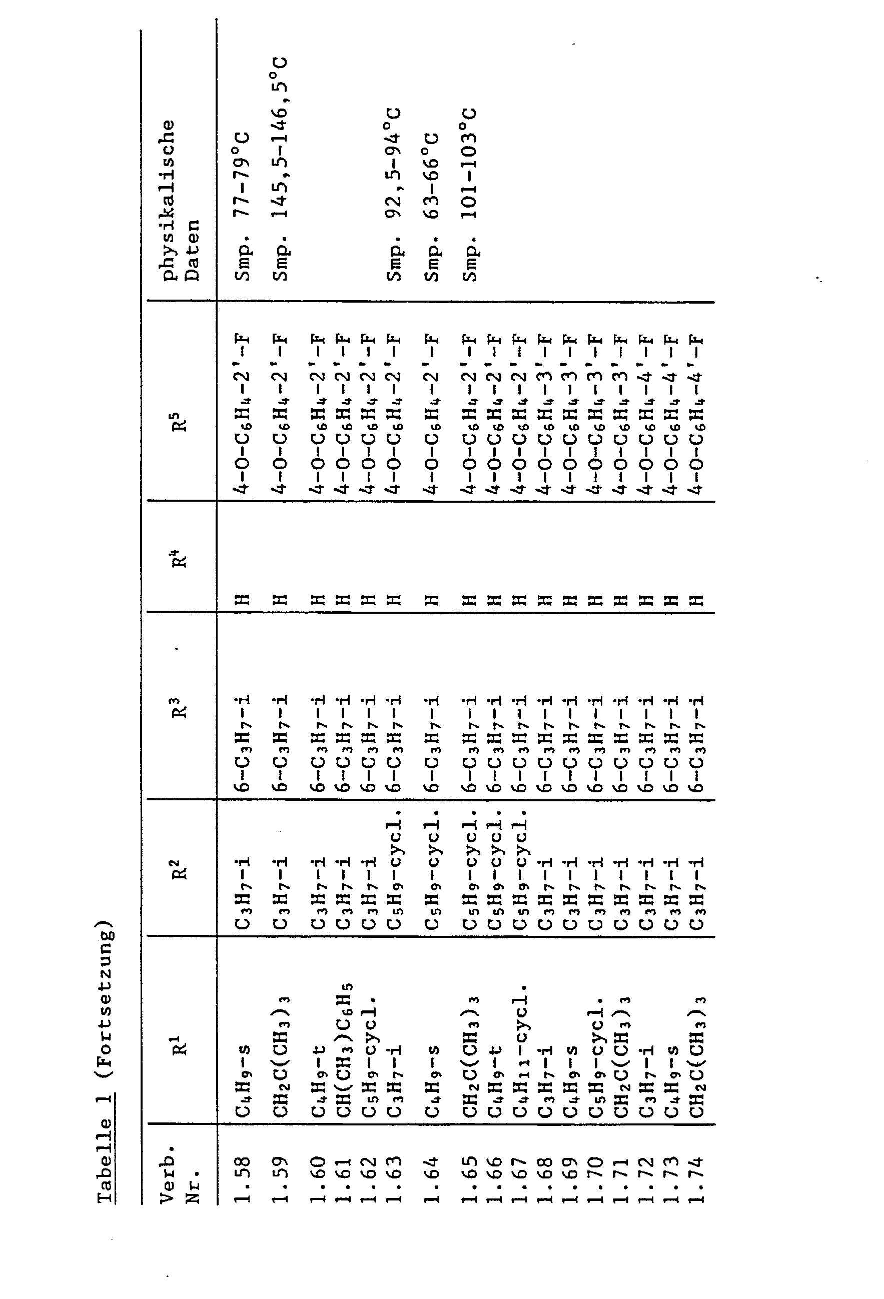

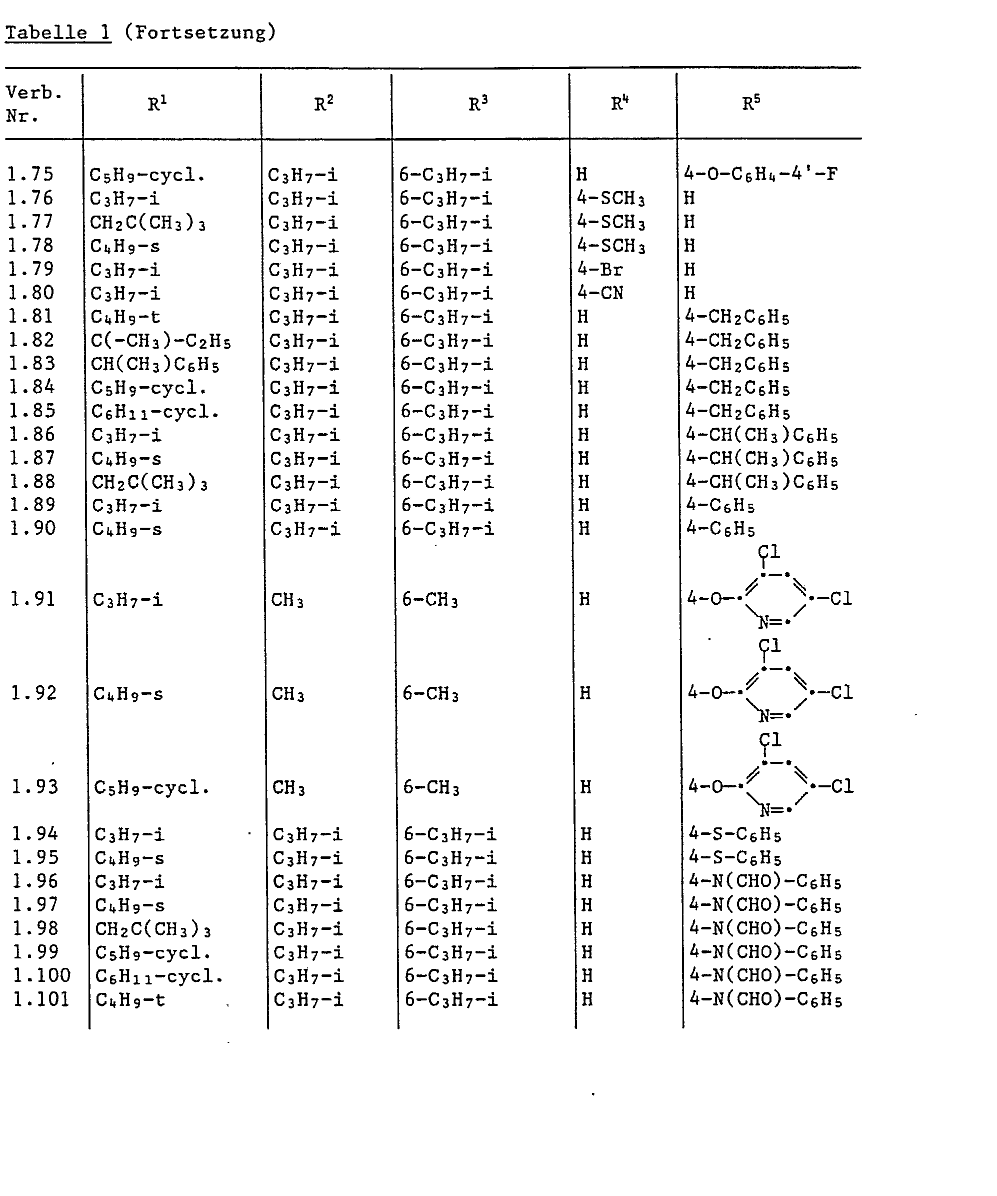

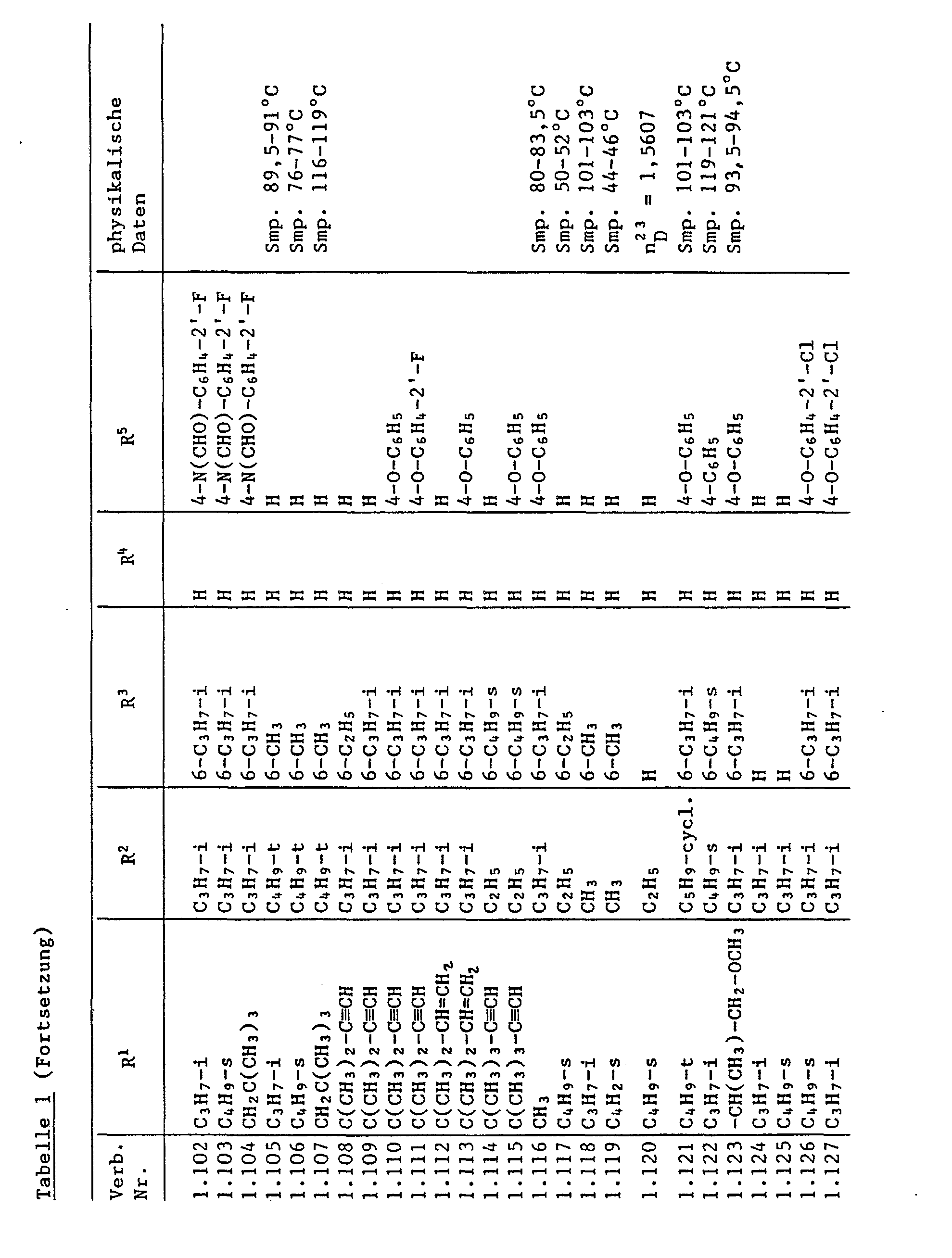

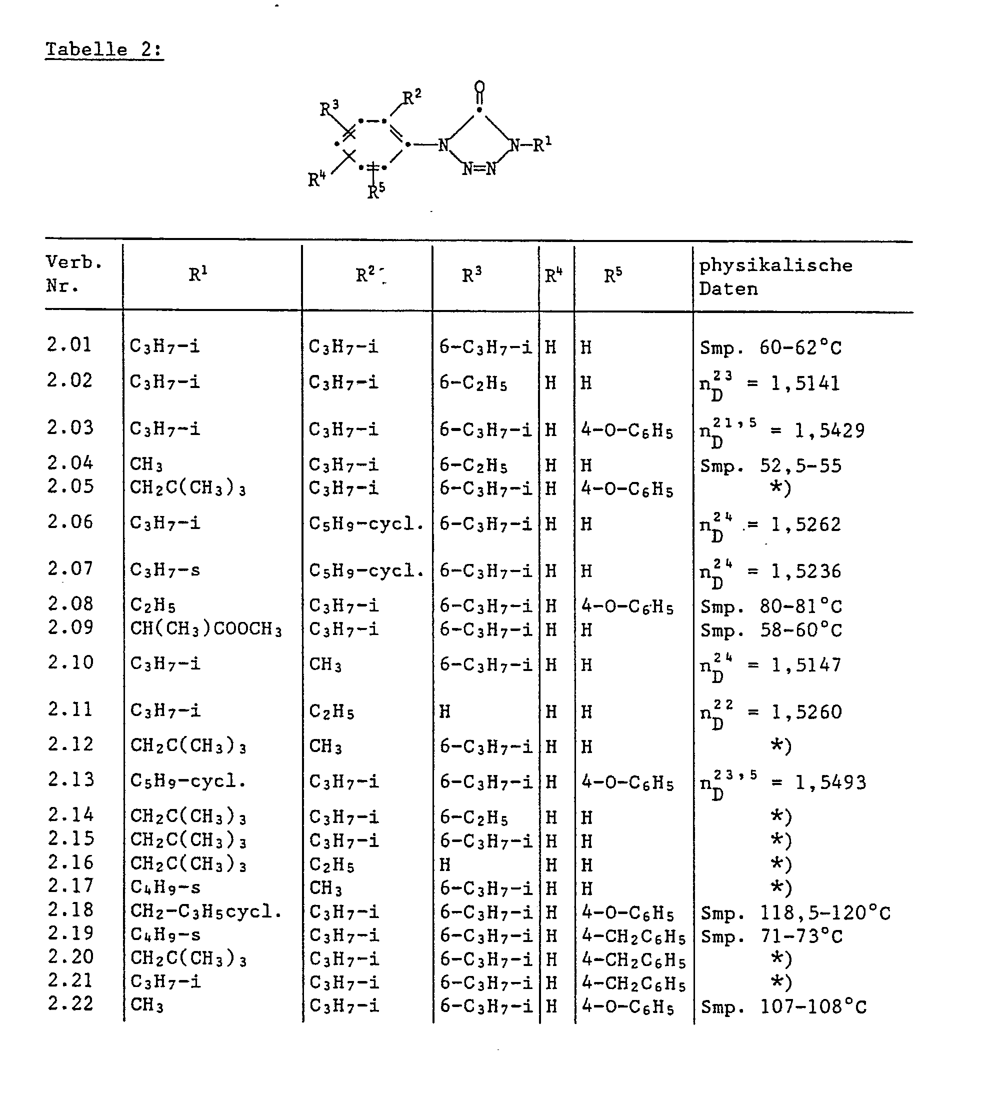

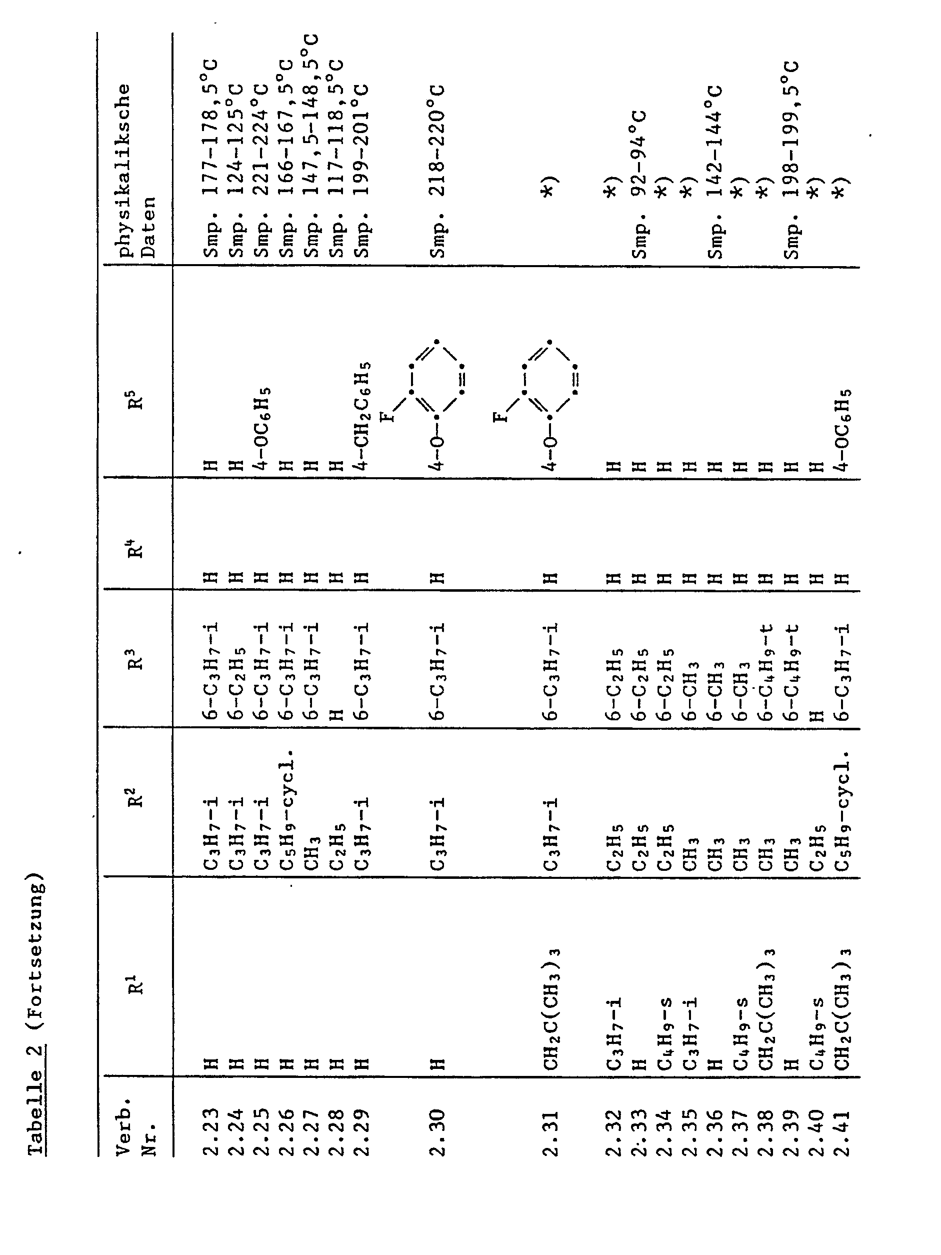

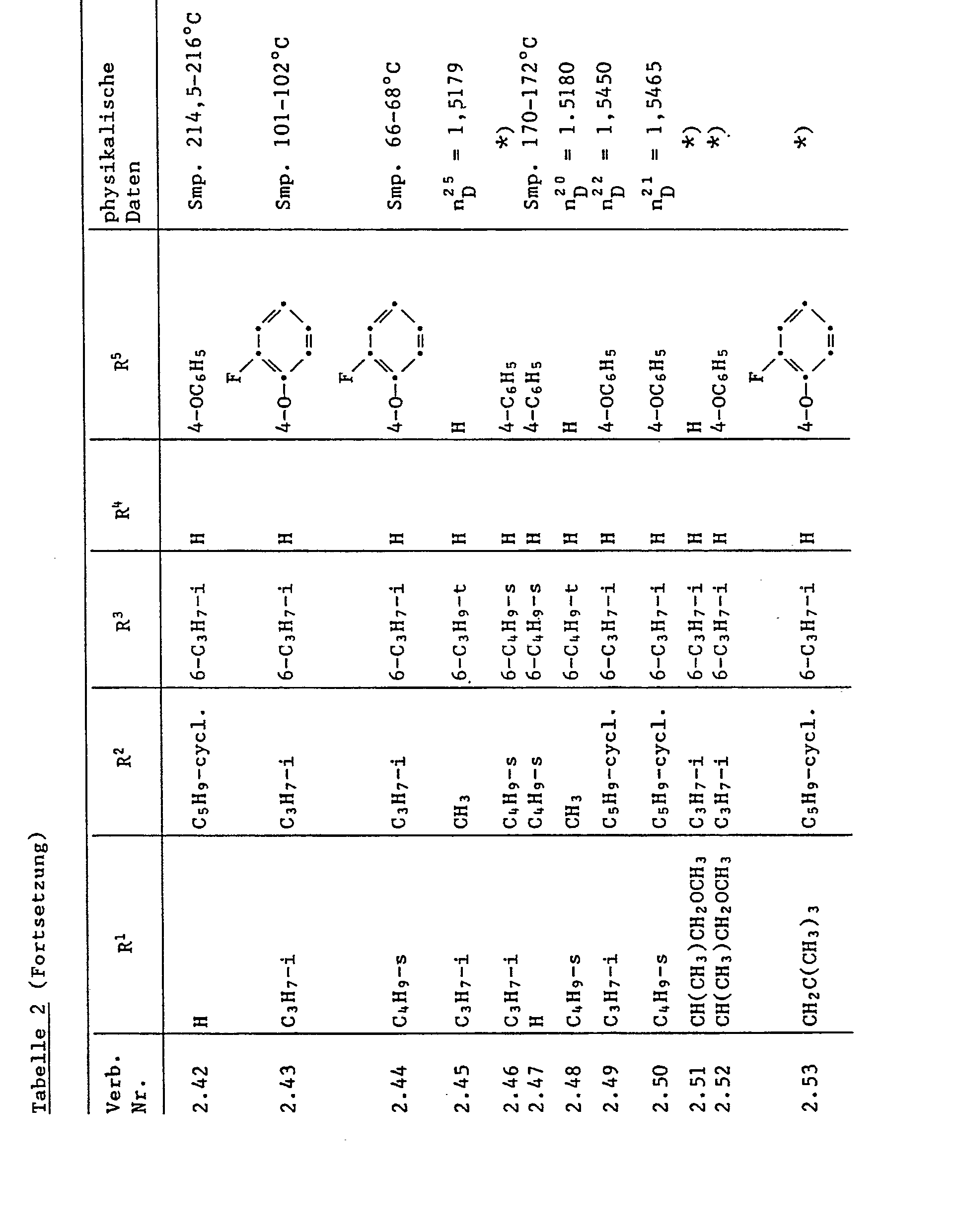

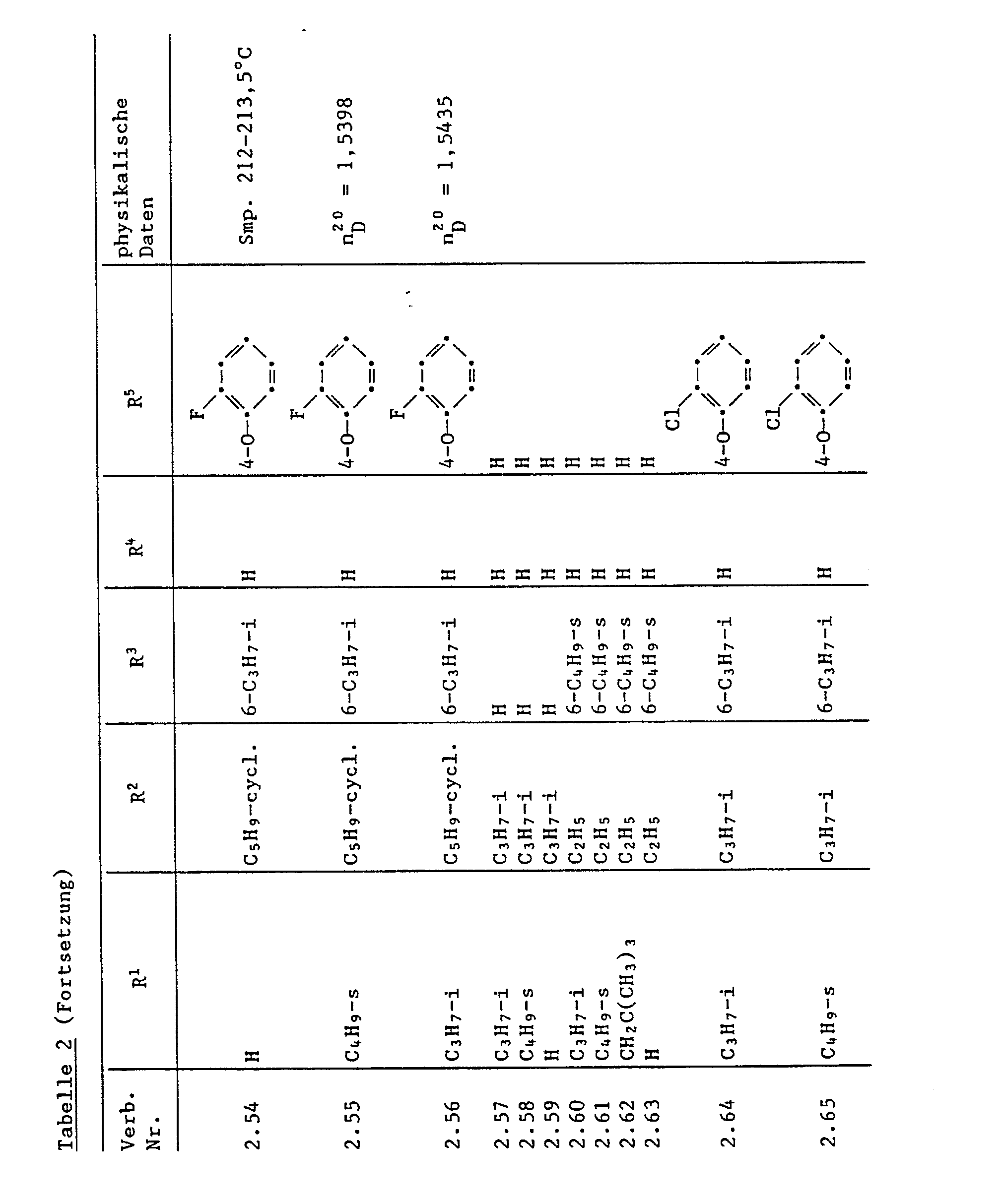

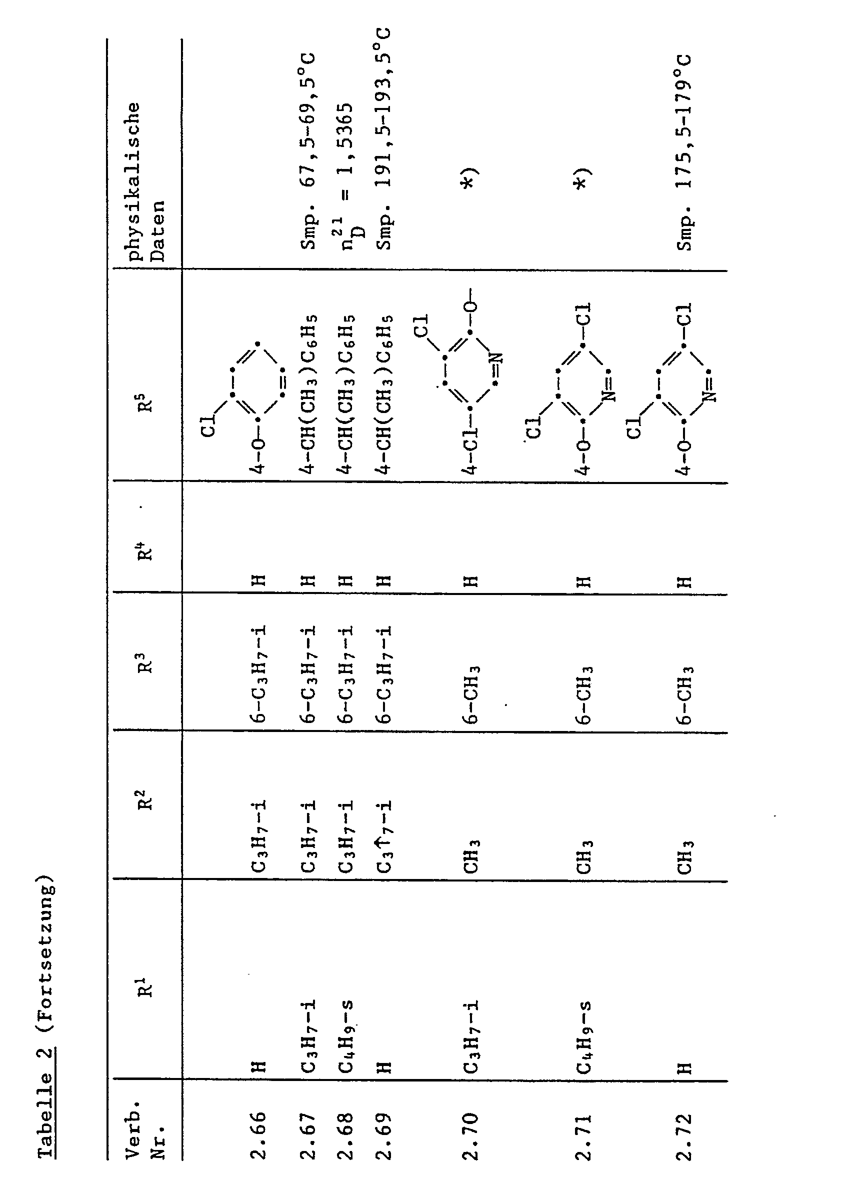

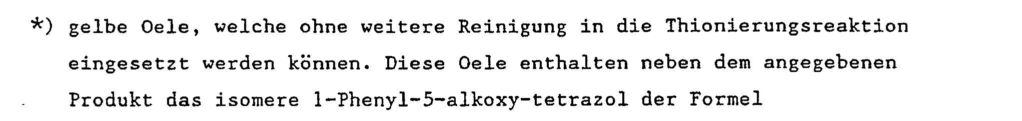

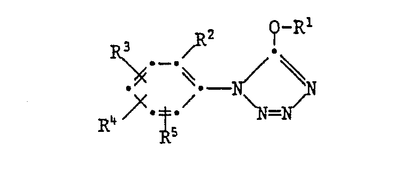

### Formulierungsbeispiele (% = Gewichtsprozent)

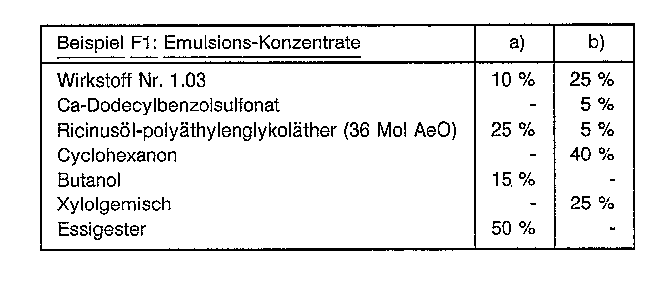

Aus solchen Konzentraten können durch Verdünnen mit Wasser Emulsionen jeder gewünschten Konzentration hergestellt werden.

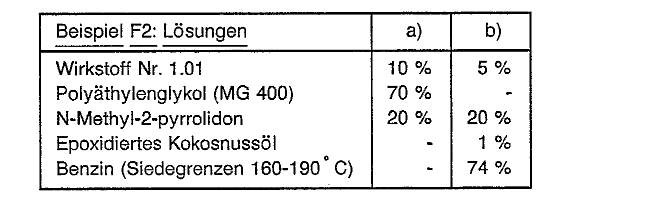

Die Lösungen sind zur Anwendung in Form kleinster Tropfen geeignet.

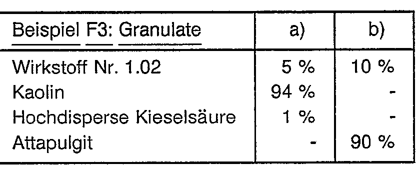

Der Wirkstoff wird in Methylenchlorid gelöst, auf den Träger aufgesprüht und das Lösungsmittel anschliessend im Vakuum abgedampft.

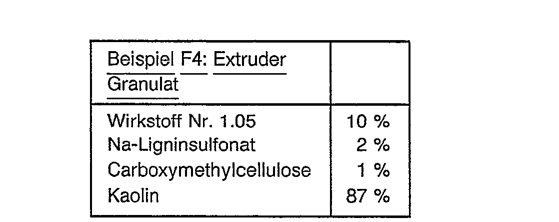

Der Wirkstoff wird mit den Zusatzstoffen vermischt, vermahlen und mit Wasser angefeuchtet. Dieses Gemisch wird extrudiert und anschliessend im Luftstrom getrocknet.

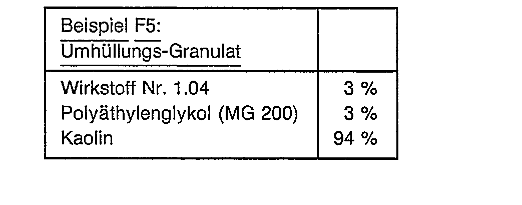

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

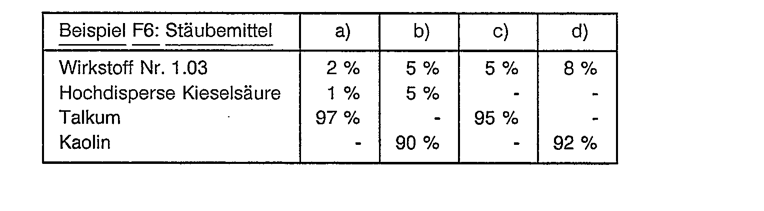

Durch inniges Vermischen der Trägerstoffe mit dem Wirkstoff und gegebenenfalls Vermahlen auf einer geeigneten Mühle erhält man gebrauchsfertige Stäubemittel.

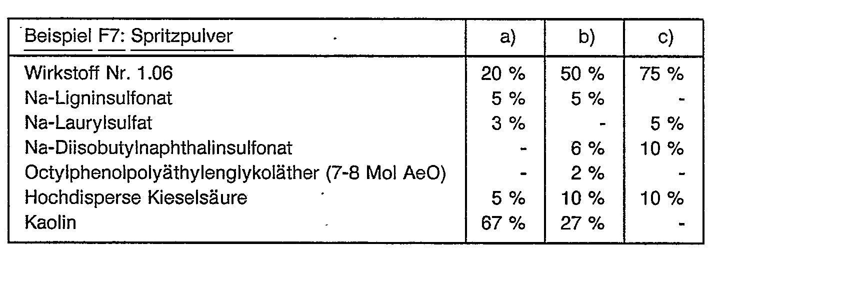

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

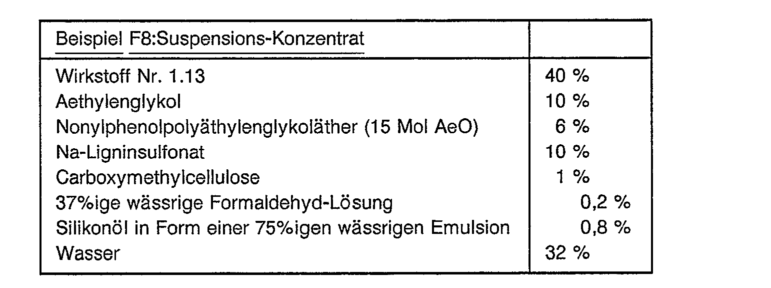

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele

### Beispiel B1: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Verbindungen aus der Tabelle 1 zeigen eine gute Wirkung gegen Diabrotica balteata in diesem Test. Die Verbindungen 1.01, 1.02, 1.03, 1.05, 1.10, 1.12, 1.33, 1.30, 1.18, 1.31, 1.59, 1.14, 1.107, 1.57, 1.58, 1.105, 1.106 und 1.52 zeigen eine Wirkung von mehr als 80 %.

### Beispiel B2: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25 * C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Verbindungen aus der Tabelle 1 zeigen eine gute Wirkung gegen Tetranychus urticae in diesem Test. Die Verbindungen 1.03, 1.05, 1.07, 1.13, 1.33, 1.30, 1.31, 1.29, 1.57, 1.105 und 1.52 reduzieren die Population um mehr als 80 %.

### Beispiel B3: Wirkung gegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frassschadens auf den behandelten zu denjenigen auf den unbehandelten Pflanzen wird die prozentuale Reduktion der Population bzw. die prozentuale Reduktion des Frassschaden (% Wirkung) bestimmt.

Verbindungen aus der Tabelle 1 zeigen eine gute Wirkung gegen Spodoptera littoralis in diesem Test. Die Verbindung 1.03 bewirkt auch bei 50 ppm eine Populationreduktion von mehr als 80 %.

### Beispiel B4: Wirkung gegen Crocidolomia binotalis Raupen

Junge Kohlpflanzen werden mit einer wässrigen Emulsions-Spritzbrühe, die 400 ppm des Wirkstoffes enthält, besprüht. Nach dem Antrocknen des Spritzbelages werden die Kohlpflanzen mit 10 Raupen des dritten Stadiums von Crocidolomia binotalis besiedelt und in einen Plastikbehälter gegeben. 3 Tage später erfolgt die Auswertung. Aus dem Vergleich der Anzahl toter Raupen und des Frassschadens auf den behandelten zu denjenigen auf den unbehandelten Pflanzen wird die prozentuale Reduktion der Population bzw. die prozentuale Reduktion des Frassschaden (% Wirkung) bestimmt.

Verbindungen aus der Tabelle 1 zeigen eine gute Wirkung gegen Crocidolomia binotalis in diesem Test. Die Verbindung 1.03 ist auch bei 50 ppm zu mehr als 80 % wirksam.

### Beispiel B5: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten des Anthonomus grandis besiedelt und in einen Plastikbehälter gegeben. 3 Tage später erfolgt die Auswertung. Aus dem Vergleich der Anzahl toter Käfer und des Frassschadens auf den behandelten zu denjenigen auf den unbehandelten Pflanzen wird die prozentuale Reduktion der Population bzw. die prozentuale Reduktion des Frassschaden (% Wirkung) bestimmt.

Verbindungen aus der Tabelle 1 zeigen eine gute Wirkung gegen Anthonomus grandis in diesem Test. Die Verbindung 1.03 erzeugt auch bei 200 ppm noch eine Wirkung von mehr als 80 %.

### Beispiel B6: Systemische Wirkung auf Nilaparvata lugens

Etwa 10 Tage alte Reispflanzen werden in einen Plastik-Becher eingesetzt, der 20 ml einer wässrigen Emulsions-Zubereitung des zu prüfenden Wirkstoffes in einer Konzentration von 50 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Dann wird die Reispflanze mit 20 Nymphen von Nilaparvata lugens im N₂- bis N₃-Stadium besiedelt und mit einem Plastikzylinder abgedeckt. Der Versuch wird bei 26 C und ca. 60 % relativer Luftfeuchtigkeit mit einer Beleuchtungsperiode von 16 Stunden durchgeführt. Nach zwei und fünf Tagen wird auf die Anzahl der abgetöteten Testtiere, im Vergleich zu unbehandelten Kontrollen, festgestellt.

Verbindungen aus der Tabelle 1 zeigen im obigen Test eine gute Wirkung gegen Nilaparvata lugens. Die Verbindungen 1.01, 1.02, 1.03, 1.04, 1.06, 1.07, 1.10, 1.12, 1.18, 1.29, 1.14, 1.107, 1.105 und 1.106 haben eine 80-100 %ige Wirkung.

### Beispiel B7: Wirkung gegen Nilaparvata lugens

Reispflanzen werden mit einer wässrigen Emulsions-Spritzbrühe, die 400 ppm des Wirkstoffes enthält, behandelt. Nach Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen aus der Tabelle 1 zeigen eine gute Wirkung gegen Nilaparvata lugens in diesem Test. Insbesondere die Verbindungen 1.01, 1.02, 1.03, 1.06, 1.05, 1.08, 1.07, 1.13, 1.12, 1.33, 1.30, 1.18, 1.32, 1.29, 1.27, 1.14, 1.107, 1.57, 1.58, 1.105 und 1.106 zeigen eine Wirkung über 80 %

### Beispiel B8: Wirkung gegen Nephotettix cincticeps

Reispflanzen werden mit einer wässrigen Emulsions-Spritzbrühe, die 400 ppm des Wirkstoffes enthält, behandelt. Nach Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen aus der Tabelle 1 zeigen eine gute Wirkung gegen Nephotettix cincticeps in diesem Test. Insbesondere der Verbindungen 1.01, 1.03, 1.05, 1.08, 1.33, 1.107, 1.57, 1.58, 1.105 und 1.106 zeigen eine 80-100 %ige Wirkung.

### Beispiel B9: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20 C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (%-Wirkung) bestimmt.

Verbindungen aus der Tabelle 1 zeigen eine gute Wirkung gegen Aphis craccivora in diesem Test. Insbesondere die Verbindungen 1.01, 1.02, 1.07, 1.11, 1.14 zeigen eine Wirkung über 80 %.

### Beispiel B10: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält, übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Die Verbindungen gemäss Tabelle 1 zeigen in diesem Test gute Wirkung gegen Boophilus microplus. Insbesondere die Verbindungen 1.01, 1.02, 1.03, 1.05, 1.08, 1.13, 1.18, 1.30, 1.38, 1.33 und 1.59 zeigen eine Wirkung über 80 %.

### Beispiel B11: Wirkung gegen Musca domestica

Ein Zuckerwürfel wird mit einer Lösung der Testsubstanz so behandelt, dass die Konzentration von Testsubstanz, nach Trocknen über Nacht, im Zucker 250 ppm beträgt. Dieser behandelte Würfel wird mit einem nassen Wattebausch und 10 adulten (ca. 1 Woche alten) Fliegen [Spezies M. domestica; Stamm . Schmidlin (Organophosphat-resistent)] auf eine Alluminiumschale gelegt. Das Ganze wird mit einem Becherglas abgedeckt und während 24 Stunden bei 25 C und 50 % Luftfeuchtigkeit belassen. Nach Ablauf dieser Frist wird die Mortalitätsrate bestimmt.

Die Verbindungen der Tabelle 1 zeigen in diesem Test eine gute Wirkung gegen Musca domestica. Insbesondere die Verbindungen 1.08, 1.30 und 1.58 zeigen eine Wirkung über 80 %.

### Beispiel B12: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Verbindungen aus der Tabelle 1 zeigen eine gute Wirkung gegen Heliothis virescens in diesem Test. Insbesondere die Verbindungen 1.33 und 1.31 zeigen eine Wirkung über 80 %.

## Patentansprüche

1. 1-Phenyl-5-thioxo-2-tetrazoline der Formel I 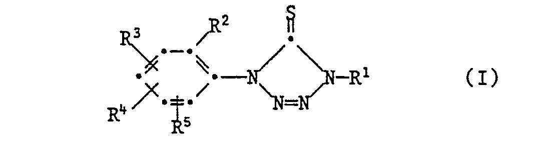 worin
R' für C₁-C₈-Alkyl, C₃-C₆-Alkenyl, C₃-C₆-Alkinyl, Cₛ-C₆-Cycloalkyl, Cₛ-C₆-Cycloalkenyl, durch C₁-C₄-Alkyl oder Halogen substituiertes C₃-C₆-Cycloalkyl, durch C₁-C₄-Alkyl oder Halogen substituiertes Cs-Cs-Cycloalkenyl, durch Halogen, C₁-C₄-Alkoxy oder Phenyl substituiertes Cₛ-C₆-Alkenyl, durch Halogen, C₁-C₄-Alkoxy oder Phenyl substituiertes C₃-C₈-Alkinyl oder durch Halogen, C₁-C₄-Alkoxy, C₁-C₄-Alkylthlo, C₁-C₄-Alkoxycarbonyl, C₃-C₆-Cycloalkyl, Phenyl, Cyan, Hydroxy, Halogenphenyl, C₁-C₄-Alkylphenyl oder einen heteroaromatischen Rest substituiertes C₁-C₈-Alkyl,
R² für C₁-C₆-Alkyl, C₂-C₆-Alkenyl, C₂-C₆-Alkinyl, C₃-C₆-Cycloalkyl, Cyclopentenyl, Cyclohexenyl oder durch Halogen, C₁-C₄-Alkoxy oder C₁-C₄-Alkylthio substituiertes C₁-C₆-Alkyl,
R³ und R⁴ unabhängig voneinander für Wasserstoff, Halogen, C₁-C₄-Alkyl, C₁-C₄-Halogenalkyl, C₁-C₄-Alkoxy, C₁-C₄-Alkylthio, C₃-C₆-Cycloalkyl, C₂-C₄-Alkenyl, C₂-C₄-Alkinyl, C₂-.C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH = CH-CH = CH-, -CH₂-CH = CH-, -(CH₂)₄-, -(CH₂)₃-, -O-CH₂-O-, -O-CH₂-CH₂-O-, -CH₂-O-CH₂-, --(CH₂)₂-CH = CH- oder -CH₂-CH = CH-CH₂-Brücke, welche durch ein bis zwei C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Alkoxy, C₁-C₄-Halogenalkoxy, C₁-C₄-Halogenalkyl, C₁-C₄-Alkylthio, C₁-C₄-Halogenalkylthio, Di-C₁-C₄-alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(Ci-C₂-Alkyl)-, -N(CHO)-, -CH₂-, -CH(CH₃)- oder -C-(CH₃)₂- bedeuten.

2. Verbindungen gemäss Anspruch 1, dadurch gekennzeichnet, dass R' für C₁-C₅-Alkyl, C₃-C₅-Alkenyl, C₃-Cₛ-Alkinyl, Cₛ-C₆-Cycloalkyl oder durch C₁-C₄-Alkoxy, Phenyl oder Cyclopropyl substituiertes C₁-C₄-Alkyl steht.

3. Verbindungen gemäss Anspruch 1, dadurch gekennzeichnet, dass R² für C,-C₄-Alkyl oder C₅-C₆-Cycloalkyl steht.

4. Verbindungen gemäss Anspruch 1, dadurch gekennzeichnet, dass R³ und R⁴ unabhängig voneinander für Wasserstoff, C₁-C₄-Alkyl, C₂-Cs-Alkinyl, C₂-C₃-Alkenyl oder C₂-C₄-Alkoxyalkyl stehen.

5. Verbindungen gemäss Anspruch 4, dadurch gekennzeichnet, dass einer der Substituenten R³ und R⁴ Wasserstoff bedeutet und der andere die 6-Stellung des Phenylringes besetzt.

6. Verbindungen gemäss Anspruch 1, dadurch gekennzeichnet, dass R⁵ für Wasserstoff, Benzyl, 1-Phenyläthyl, Phenoxy, Halogenphenoxy, Phenylthio, Phenyl, Dichlorpyridyloxy, N-Formylanilinyl oder N-Formylhalogenanilinyl steht.

7. Verbindungen gemäss Anspruch 6, dadurch gekennzeichnet, dass R⁵ für Wasserstoff, Benzyl, Phenoxy oder Halogenphenoxy in 4-Position des Phenylringes steht..

8. Verbindungen gemäss Anspruch 5, dadurch gekennzeichnet, dass R³ für C₁-C₄-Alkyl in 6-Position des Phenylringes steht und R⁴ Wasserstoff bedeutet.

9. Verbindungen gemäss Anspruch 1, dadurch gekennzeichnet, dass R² für C₁-C₄-Alkyl oder Cs-C₆-Cycloalkyl, R³ für C₁-C₄-Alkyl in 6-Position des Phenylringes, R⁴ für Wasserstoff und R⁵ für Wasserstoff, Benzyl, Phenoxy oder Halogenphenoxy in 4-Position des Phenylringes stehen.

10. Verbindungen gemäss Anspruch 1, dadurch gekennzeichnet, dass R¹ für C₁-C₅-Alkyl, C₃-C₅-Alkenyl, C₃-C₅-Alkinyl, Cₛ-C₆-Cycloalkyl oder durch C₁-C₄-Alkoxy, Phenyl oder Cyclopropyl substituiertes C₁-C₄Alkyl, R² für C₁-C₄-Alkyl oder C₅-C₆-cycloalkyl, R³ und R⁴ unabhängig für Wasserstoff, C₁-C₄-Alkyl, C₂-C₃-Alkinyl, C₂-C₃-Alkenyl oder C₂-C₄-Alkoxyalkyl und R⁵ für Wasserstoff, Benzyl, 1-Phenyläthyl, Phenoxy, Halogenphenoxy, Phenylthio, Phenyl, Dichlorpyridyloxy, N-Formylanilinyl oder N-Formylhalogenanilinyl stehen.

11. Verbindungen gemäss Anspruch 1, dadurch gekennzeichnet, dass R¹ für C₁-C₅-Alkyl oder Cₛ-Cs-Cycloalkyl, R² für C₁-C₄-Alkyl oder C₅-C₆-Cycloalkyl, R³ für C₁-C₄-Alkyl in 6-Position des Phenylringes, R⁴ für Wasserstoff, R⁵ für Wasserstoff, Benzyl, Phenoxy oder Halogenphenoxy in 4-Position des Phenylringes stehen.

12. Verbindungen gemäss Anspruch 1, ausgewählt aus der Gruppe 1-(2-Aethyl-6-isopropylphenyl)-4-isopropyl-5-thioxo-2-tetrazolin, 1-(2,6-Diisopropyl-4-phenoxyphenyl)-4-isopropyl-5-thioxo-2-tetrazolin, 1-(2-Aethyl-6-isopropylphenyl)-4-methyl-5-thioxo-2-tetrazolin, 1-(2,6-Diisopropyl-4-phenoxyphenyl)-4-(2,2-dimethylpropyl)-5-thioxo-2-tetrazolin, 1-(2-Cyclopentyl-6-isopropylphenyl)-4-isopropyl-5-thioxo-2-tetrazolin, 1-(2-Cyclopentyl-6-isopropylphenyl)-4-sec.butyl-5-thioxo-2-tetrazolin, 1-(2,6-Diisopropyl-4-phenoxyphenyl)-4-äthyl-5-thioxo-2-tetrazolin, 1-(2,6-Diisopropylphenyl)-4-(1-methyl-2-methoxyäthyl)-5-thioxo-2-tetrazolin, 1-(2-Methyl-6-isopropylphenyl)-4-isopropyl-5-thioxo-2-tetrazolin, 1-(2-Methyl-6-isopropylphenyl)-4-(2,2-dimethylpropyl)-5-thioxo-2-tetrazolin, 1-(2,6-Diisopropyl-4-phenoxyphenyl)-4-cyclopentyl-5-thioxo-2-tetrazolin, 1-(2,6-Diisopropylphenyl)-4-isopropyl-5-thioxo-2-tetrazolin, 1-(2-Aethyl-6-isopropylphenyl)-4-(2,2-dimethylpropyl)-5-thioxo-2-tetrazolin, 1-(2,6-Diisopropylphenyl)-4-(2,2-dimethylpropyl)-5-thioxo-2-tetrazolin, 1-(2,6-Diisopropylphenyl)-4-(2-cyclohexenyl)-5-thioxo-2-tetrazolin, 1-(2-Methyl-6-isopropylphenyl)-4-sek.butyl-5-thioxo-2-tetrazolin, 1-(2,6-Diisopropyl-4-phenoxyphenyl)-4-cyclopropylmethyl-5-thioxo-2-tetrazolin, 1-(2,6-Diisopropyl-4-benzylphenyl)-4-sek.butyl-5-thioxo-2-tetrazolin, 1-(2,6-Diisopropyl-4-benzylphenyl)-4-(2,2-dimethylpropyl)-5-thioxo-2-tetrazolin, 1-(2,6-Diisopropyl-4-benzylphenyl)-4-isopropyl-5-thioxo-2-tetrazolin, 1-[2,6-Diisopropyl-4-(2-fluorphenoxy)-phenyl]-4-isopropyl-5-thioxo-2-tetrazolin, 1-[2,6-Diisopropyl-4-(1-phenyläthyl)-phenyl]-4-isopropyl-5-thioxo-2-tetrazolin, 1-(2-Cyclopentyl-6-isopropyl-4-phenoxyphenyl)-4-isopropyl-5-thioxo-2-tetrazolin, 1-(2-tert.Butyl-6-methylphenyl)-4-isopropyl-5-thioxo-2-tetrazolin, 1-(2-tert.Butyl-6-methylphenyl)-4-sec.butyl-5-thioxo-2-tetrazolin, 1-(2-tert.Butyl-6-methylphenyl)-4-(2,2-dimethylpropyl)-5-thioxo-2-tetrazolin und 1-(2,6-Diäthylphenyl)-4-isopropyl-5-thioxo-2-tetrazolin.

13. Verfahren zur Herstellung der Verbindungen der Formel I, dadurch gekennzeichnet, dass man entweder
a) ein 1-Phenyl-5-oxo-2-tetrazolin der Formel II 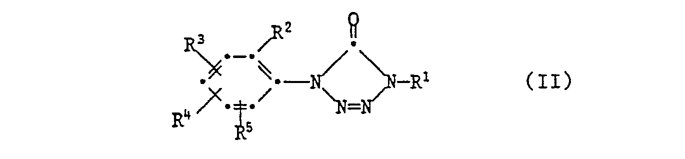 worin R¹, R², R³, R⁴ und R⁵ die unter Formel I gegebenen Bedeutungen haben, mit einem Thionierungsmittel behandelt, oder
b) ein 1-Phenyl-5-thioxo-2-tetrazolin der Formel III 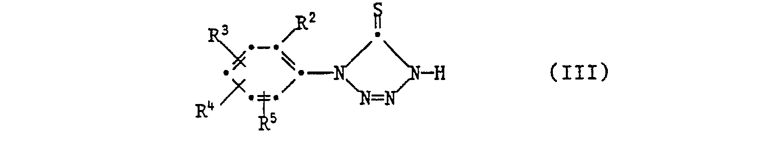 worin R², R³, R⁴ und R⁵ die unter Formel I gegebenen Bedeutungen haben, mit einem Alkylierungsmittel der Formel IV X - R" (IV) worin R" die Bedeutung von R¹ wie unter Formel I hat oder für einen in R' überführbaren Substituenten steht und X für eine Abgangsgruppe steht, alkyliert und das entstandene 1-Phenyl-5-mercaptotetrazol der Formel V 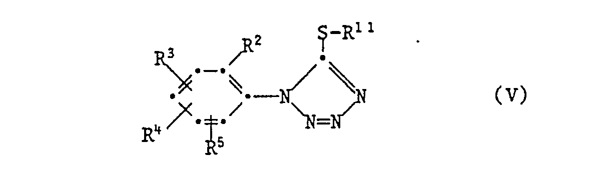 durch eine Umlagerungsreaktion in das entsprechende 1-Phenyl-5-thioxo-2-tetrazolin der Formel la 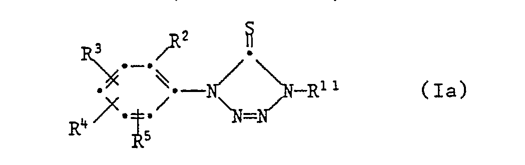 überführt und gewünschtenfalls den Substituenten R" in einen Rest gemäss der Definition von R¹ umwandelt, oder
c) ein 1-Phenyl-5-thioxo-2-tetrazolin der Formel III, in Gegenwart einer Base durch Umsatz mit einem aktivierten in den Rest R¹ überführbaren Carbonylvinyl- oder Cyanovinyl-Derivat alkyliert und den eingeführten Substituenten gewünschtenfalls in einen Rest gemäss der Definition von R¹ umwandelt.

14. Schädlingsbekämpfungsmittel, welches als aktive Komponente mindestens eine Verbindung der Formel I nach Anspruch 1 enthält.

15. Mittel gemäss Anspruch 14, dadurch gekennzeichnet, dass es zusätzlich mindestens einen Trägerstoff enthält.

16. Verwendung einer Verbindung der Formel I nach Anspruch 1 zur Bekämpfung von Schädlingen an Tieren und Pflanzen.

17. Verwendung gemäss Anspruch 16, dadurch gekennzeichnet, dass es sich bei den Schädlingen um pflanzenschädigende Insekten und Arachniden handelt.

18. Verfahren zur Bekämpfung von tier- und pflanzenschädigenden Insekten und Arachniden, dadurch gekennzeichnet, dass man die Pflanzen oder ihren Lebensraum mit einer wirksamen Nenge einer Verbindung der Formel I gemäss Anspruch 1 behandelt.

19. 1-Phenyl-5-thioxo-2-tetrazoline der Formel II 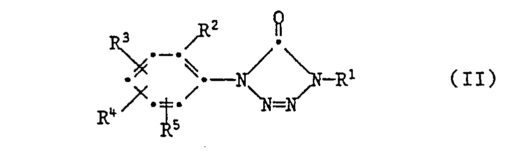 worin
R¹ für C₁-C₈-Alkyl, C₃-C₆-Alkenyl, C₃-C₆-Alkinyl, C₃-C₆-Cycloalkyl, Cₛ-C₆-Cycloalkenyl, durch C₁-C₄-Alkyl oder Halogen substituiertes C₃-C₆-Cycloalkyl, durch C₁-C₄-Alkyl oder Halogen substituiertes C₅-C₆-Cycloalkenyl, durch Halogen, C₁-C₄-Alkoxy oder Phenyl substituiertes Cs-C₆-Alkenyl, durch Halogen, C₁-C₄-Alkoxy oder Phenyl substituiertes Cs-Cs-Alkinyl oder durch Halogen, C₁-C₄-Alkoxy, C₁-C₄-Alkylthio, C₁-C₄-Alkoxycarbonyl, C₃-C₆-Cycloalkyl, Phenyl, Cyan, Hydroxy, Halogenphenyl, Ci-C₄-Alkylphenyl oder einen heteroaromatischen Rest substituiertes C₁-C₈-Alkyl,
R² für C₁-C₆-Alkyl, C₂-C₆-Alkenyl, C₂-C₆-Alkinyl, C₃-C₆-Cycloalkyl, Cyclopentenyl, Cyclohexenyl oder durch Halogen, C₁-C₄-Alkoxy oder C₁-C₄-Alkylthio substituiertes C₁-C₆-Alkyl,
R³ und R⁴ unabhängig voneinander für Wasserstoff, Halogen, Cₗ-C₄-Alkyl, Ci-C₄-Halogenalkyl, C₁-C₄-Alkoxy, C₁-C₄-Alkylthio, C₃-C₆-Cycloalkyl, C₂-C₄-Alkenyl, C₂-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH = CH-CH = CH-, -CH₂-CH = CH-, -(CH₂)₄-, -(CH₂)₃-, -0-CH₂-0-, -O-CH₂-CH₂-O-, -CH₂-0-CH₂-, --(CH₂)₂-CH = CH- oder -CH₂-CH = CH-CH₂-Brücke, welche durch ein bis zwei C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl,
Ci-C₄-Alkoxy, C₁-C₄-Halogenalkoxy, C₁-C₄-Halogenalkyl, Cₗ-C₄-Alkylthio, Ci-C₄-Halogenalkylthio, Di-C₁-C₄-alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(Ci-C₂-Alkyl)-, -N(CHO)-, -CH₂-, -CH(CH₃)- oder -C-(CH₃)₂- bedeuten.

20. 1-Phenyl-5-oxo-2-tetrazoline der Formel VIII 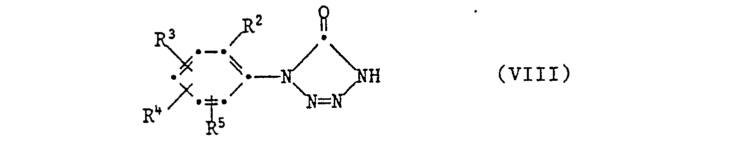 worin
R² für C₁-C₆-Alkyl, C₂-C₆-Alkenyl, C₂-C₆-Alkinyl, C₃-C₆-Cycloalkyl, Cyclopentenyl, Cyclohexenyl oder durch Halogen, C₁-C₄-Alkoxy oder C₁-C₄-Alkylthio substituiertes C₁-C₆-Alkyl,
R³ und R⁴ unabhängig voneinander für Wasserstoff, Halogen, C₁-C₄-Alkyl, C₁-C₄-Halogenalkyl, C₁-C₄-Alkoxy, C₁-C₄-Akylthio, C₃-C₆-Cycloalkyl, C₂-C₄-Alkenyl, C₂-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH = CH-CH = CH-, -CH₂-CH = CH-, -(CH₂)₄-, -(CH₂)₃-, -O-CH₂-O-, -O-CH₂-CH₂-O-, -CH₂-O-CH₂-, --(CH₂)₂-CH = CH- oder -CH₂-CH = CH-CH₂-Brücke, welche durch ein bis zwie C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Alkoxy, C₁-C₄-Halogenalkoxy, C₁-C₄-Halogenalkyl, C₁-C₄-Alkylthio, C₁-C₄-Halogenalkylthio, Di-C₁-C₄-alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(C₁-C₂-Alkyl)-, -N(CHO)-, -CH₂-, -CH(CH₃)- oder -C-(CH₃)₂- bedeuten.

Patentansprüche für folgende Vertragsstaat : ES

1. Schädlingsbekämpfungsmittel, welches als aktive Komponente mindestens ein 1-Phenyl-5-thioxo-2-tetrazolin der Formel I 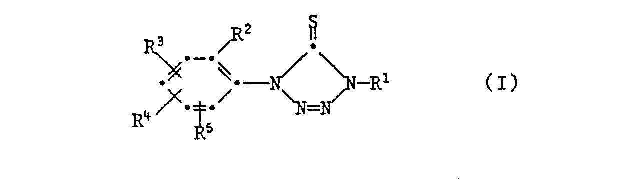 enthält, worin
R¹ für C₁-C₈-Alkyl, C₃-C₆-Alkenyl, Cs-C₆-Alkinyl, C₃-C₆-Cycloalkyl, Cs-C₆-Cycloalkenyl, durch C₁-C₄-Alkyl oder Halogen substituiertes Cₛ-C₆-Cycloalkyl, durch C₁-C₄-Alkyl oder Halogen substituiertes C₅-C₆-Cycloalkenyl, durch Halogen, C₁-C₄-Alkoxy oder Phenyl substituiertes Cs-C₆-Alkenyl, durch Halogen, C₁-C₄-Alkoxy oder Phenyl substituiertes C₃-C₈-Alkinyl oder durch Halogen, C₁-C₄-Alkoxy, C₁-C₄-Alkylthio, C₁-C₄-Alkoxycarbonyl, C₃-C₆-Cycloalkyl, Phenyl, Cyan, Hydroxy, Halogenphenyl, C₁-C₄-Alkylphenyl oder einen heteroaromatischen Rest substituiertes C₁-C₈-Alkyl,
R² für C₁-C₆-Alkyl, C₂-C₆-Alkenyl, C₂-C₆-Alkinyl, C₃-C₆-Cycloalkyl, Cyclopentenyl, Cyclohexenyl oder durch Halogen, C₁-C₄-Alkoxy oder C₁-C₄-Alkylthio substituiertes C₁-C₆-Alkyl,
R³ und R⁴ unabhängig voneinander für Wasserstoff, Halogen, C₁-C₄-Alkyl, C₁-C₄-Halogenalkyl, C₁-C₄-Alkoxy, C₁-C₄-Alkylthio, C₃-C₆-Cycloalkyl, C₂-C₄-Alkenyl, C₂-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH = CH-CH = CH-, -CH₂-CH = CH-, -(CH₂)₄-, -(CH₂)₃-, -O-CH₂-O-, -O-CH₂-CH₂-O-, -CH₂-O-CH₂-, --(CH₂)₂-CH = CH- oder -CH₂-CH = CH-CH₂-Brücke, welche durch ein bis zwei C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Alkoxy, C₁-C₄-Halogenalkoxy, C₁-C₄-Halogenalkyl, Ci-C₄-Alkylthio, Ci-C₄-Halogenalkylthio, Di-Ci-C₄-alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(C₁-C₂-Alkyl)-, -N(CHO)-, -CH₂-, -CH(CH₃)- oder -C-(CH₃)₂- bedeuten.

2. Mittel gemäss Anspruch 1, dadurch gekennzeichnet, dass R' für C₁-C₅-Alkyl, Cs-Cs-Alkenyl, Cs-Cs-Alkinyl, Cₛ-C₆-Cycloalkyl oder durch C₁-C₄-Alkoxy, Phenyl oder Cyclopropyl substituiertes C₁-C₄-Alkyl steht.

3. Mittel gemäss Anspruch 1, dadurch gekennzeichnet, dass R² für Cₗ-C₄-Alkyl oder Cₛ-C₆-Cycloalkyl steht.

4. Mittel gemäss Anspruch 1, dadurch gekennzeichnet, dass R³ und R⁴ unabhängig voneinander für Wasserstoff, C₁-C₄-Alkyl, C₂-C₃-Alkinyl, C₂-C₃-Alkenyl oder C₂-C₄-Alkoxyalkyl stehen.

5. Mittel gemäss Anspruch 4, dadurch gekennzeichnet, dass einer der Substituenten R³ und R4- Wasserstoff bedeutet und der andere die 6-Stellung des Phenylringes besetzt.

6. Mittel gemäss Anspruch 1, dadurch gekennzeichnet, dass R⁵ für Wasserstoff, Benzyl, 1-Phenyläthyl, Phenoxy, Halogenphenoxy, Phenylthio, Phenyl, Dichlorpyridyloxy, N-Formylanilinyl oder N-Formylhalogenanilinyl steht.

7. Mittel gemäss Anspruch 6, dadurch gekennzeichnet, dass R⁵ für Wasserstoff, Benzyl, Phenoxy oder Halogenphenoxy in 4-Position des Phenylringes steht.

8. Mittel gemäss Anspruch 5, dadurch gekennzeichnet, dass R³ für C₁-C₄-Alkyl in 6-Position des Phenylringes steht und R⁴ Wasserstoff bedeutet.

9. Mittel gemäss Anspruch 1, dadurch gekennzeichnet, dass R² für C₁-C₄-Alkyl oder C₅-C₆-Cycloalkyl, R³ für C₁-C₄-Alkyl in 6-Position des Phenylringes, R⁴ für Wasserstoff und R⁵ für Wasserstoff, Benzyl, Phenoxy oder Halogenphenoxy in 4-Position des Phenylringes stehen.

10. Mittel gemäss Anspruch 1, dadurch gekennzeichnet, dass R¹ für C₁-C₅-Alkyl, C₃-C₅-Alkenyl, Cs-Cs-Alkinyl, Cₛ-C₆-Cycloalkyl oder durch C₁-C₄-Alkoxy, Phenyl oder Cyclopropyl substituiertes C₁-C₄-Alkyl, R² für Cₗ-C₄-Alkyl oder Cₛ-C₆-Cycloalkyl, R³ und R⁴ unabhängig für Wasserstoff, C₁-C₄-Alkyl, C₂-C₃-Alkinyl, C₂-C₃-Alkenyl oder C₂-C₄-Alkoxyalkyl und R⁵ für Wasserstoff, Benzyl, 1-Phenyläthyl, Phenoxy, Halogenphenoxy, Phenylthio, Phenyl, Dichlorpyridyloxy, N-Formylanilinyl oder N-Formylhalogenanilinyl stehen.

11. Mittel gemäss Anspruch 1, dadurch gekennzeichnet, dass R¹ für C₁-C₅-Alkyl oder Cₛ-C₆-Cycloalkyl, R² für C₁-C₄-Alkyl oder Cₛ-C₆-Cycloalkyl, R³ für C₁-C₄-Alkyl in 6-Position des Phenylringes, R⁴ für Wasserstoff, R⁵ für Wasserstoff, Benzyl, Phenoxy oder Halogenphenoxy in 4-Position des Phenylringes stehen.

12. Mittel gemäss Anspruch 1, enthaltend einen Wirkstoff ausgewählt aus der Gruppe 1-(2-Aethyl-6-isopropylphenyl)-4-isopropyl-5-thioxo-2-tetrazolin, 1-(2,6-Diisopropyl-4-phenoxyphenyl)-4-isopropyl-5-thioxo-2-tetrazolin, 1-(2-Aethyl-6-isopropylphenyl)-4-methyl-5-thioxo-2-tetrazolin,
1-(2,6-Diisopropyl-4-phenoxyphenyl)-4-(2,2-dimethylpropyl)-5-thioxo-2-tetrazolin, 1-(2-Cyclopentyl-6-isopropylphenyl)-4-isopropyl-5-thioxo-2-tetrazolin,
1-(2-Cyclopentyl-6-isopropylphenyl)-4-sec.butyl-5-thioxo-2-tetrazolin,
1-(2,6-Diisopropyl-4-phenoxyphenyl)-4-äthyl-5-thioxo-2-tetrazolin,
1-(2,6-Diisopropylphenyl)-4-(1-methyl-2-methoxyäthyl)-5-thioxo-2-tetrazolin, 1-(2-Methyl-6-isopropylphenyl)-4-isopropyl-5-thioxo-2-tetrazolin,
1-(2-Methy(-6-isopropylphenyl)-4-(2,2-dimethylpropyl)-5-thioxo-2-tetrazolin, 1-(2,6-Diisopropyl-4-phenoxyphenyl)-4-cyclopentyl-5-thioxo-2-tetrazolin,
1-(2,6-Diisopropylphenyl)-4-isopropyl-5-thioxo-2-tetrazolin,
1-(2-Aethyl-6-isopropylphenyl)-4-(2,2-dimethylpropyl)-5-thioxo-2-tetrazolin, 1-(2,6-Diisopropylphenyl)-4-(2,2-dimethylpropyl)-5-thioxo-2-tetrazolin,
1-(2,6-Diisopropylphenyl)-4-(2-cyclohexenyl)-5-thioxo-2-tetrazolin,
1-(2-Methyl-6-isopropylphenyl)-4-sek.butyl-5-thioxo-2-tetrazolin,
1-(2,6-Diisopropyl-4-phenoxyphenyl)-4-cyclopropylmethyl-5-thioxo-2-tetrazolin, 1-(2,6-Diisopropyl-4-benzylphenyl)-4-sek.butyl-5-thioxo-2-tetrazolin,
1-(2,6-Diisopropyl-4-benzylphenyl)-4-(2,6-dimethylpropyl)-5-thioxo-2-tetrazolin, 1-(2,6-Diisopropyl-4-benzylphenyl)-4-isopropyl-5-thioxo-2-tetrazolin,
1-[2,6-Diisopropyl-4-(2-fluorphenoxy)phenyl]-4-isopropyl-5-thioxo-2-tetrazolin,
1-[2,6-Diisopropyl-4-(1-phenyläthyl)-phenyl]-4-isopropyl-5-thioxo-2-tetrazolin,
1-(2-Cyclopentyl-6-isopropyl-4-phenoxyphenyl)-4-isopropyl-5-thioxo-2-tetrazolin,
1-(2-tert.Butyl-6-methylplenyl)-4-isopropyl-5-thioxo-2-tetrazolin,
1-(2-tert.Butyl-6-methylphenyl)-4-sec.butyl-5-thioxo-2-therazolin,
1-(2-tert.Butyl-6-methylphenyl)-4-(2,2-dimethylpropyl)-5-thioxo-2-tetrazolin und 1-(2,6-Diäthylphenyl)-4-isopropyl-5-thioxo-2-tetrazolin.

13. Verfahren zur Herstellung der Verbindungen der Formel I, gemäss Anspruch 1, dadurch gekennzeichnet, dass man entweder
a) ein 1-Phenyl-5-oxo-2-tetrazolin der Formel II 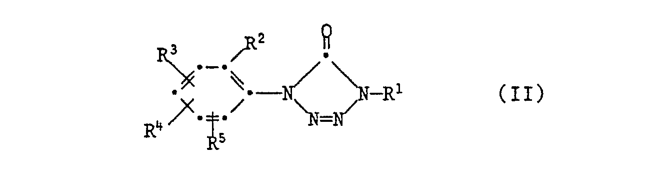 worin R¹, R², R³, R⁴ und R⁵ die unter Formel I gegebenen Bedeutungen haben, mit einem Thionierungsmittel behandelt, oder
b) ein 1-Phenyl-5-thioxo-2-tetrazolin der Formel III 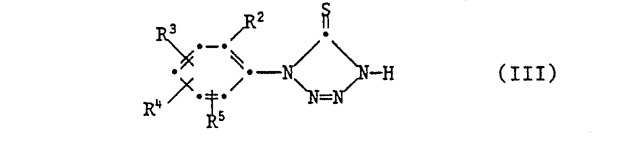 worin R², R³, R⁴ und R⁵ die unter Formel I gegebenen Bedeutungen haben, mit einem Alkylierungsmittel der Formel IV X - R" (IV) worin R¹¹ die Bedeutung von R¹ wie unter Formel I hat oder für einen in R¹ überführbaren Substituenten steht und X für eine Abgangsgruppe steht, alkyliert und das entstandene 1-Phenyl-5-mercaptotetrazol der Formel V 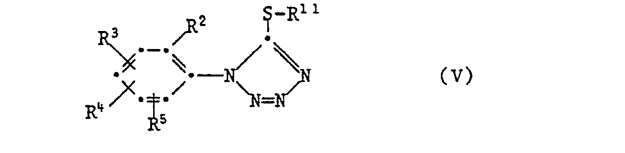 durch eine Umlagerungsreaktion in das entsprechende 1-Phenyl-5-thioxo-2-tetrazolin der Formel la 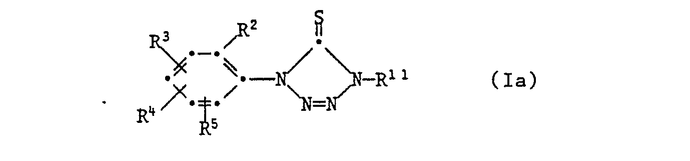 überführt und gewünschtenfalls den Substituenten R" in einen Rest gemäss der Definition von R¹ umwandelt, oder
c) ein 1-Phenyl-5-thioxo-2-tetrazolin der Formel III, in Gegenwart einer Base durch Umsatz mit einem aktivierten in den Rest R¹ überführbaren Carbonylvinyl- oder Cyanovinyl-Derivat alkyliert und den eingeführten Substituenten gewünschtenfalls in einen Rest gemäss der Definition von R¹ umwandelt.

14. Mittel gemäss Anspruch 1, dadurch gekennzeichnet, dass es zusätzlich mindestens einen Trägerstoff enthält.

15. Verwendung einer Verbindung der Formel I nach Anspruch 1 zur Bekämpfung von Schädlingen an Tieren und Pflanzen.

16. Verwendung gemäss Anspruch 15, dadurch gekennzeichnet, dass es sich bei den Schädlingen um pflanzenschädigende Insekten und Arachniden handelt.

17. Verfahren zur Bekämpfung von tier- und pflanzenschädigenden Insekten und Arachniden, dadurch gekennzeichnet, dass man die Pflanzen oder ihren Lebensraum mit einer wirksamen Menge einer Verbindung der Formel I gemäss Anspruch 1 behandelt.

18. Verfahren zur Herstellung von 1-Phenyl-5-thioxo-2-tetrazoline der Formel II 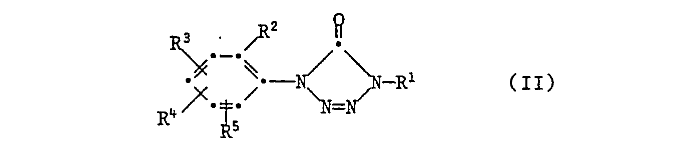 worin
R¹ für C₁-C₈-Alkyl, Cₛ-C₆-Alkenyl, C₃-C₆-Alkinyl, C₃-C₆-Cycloalkyl, Cₛ-C₆-Cycloalkenyl, durch C₁-C₄-Alkyl oder Halogen substituiertes C₃-C₆-Cycloalkyl, durch C₁-C₄-Alkyl oder Halogen substituiertes Cₛ-C₆-Cycloalkenyl, durch Halogen, C₁-C₄-Alkoxy oder Phenyl substituiertes C₃-C₆-Alkenyl, durch Halogen, C₁-C₄-Alkoxy oder Phenyl substituiertes Cₛ-Cs-Alkinyl oder durch Halogen, C₁-C₄-Alkoxy, C₁-C₄-Alkylthio, C₁-C₄-Alkoxycarbonyl, C₃-C₆-Cycloalkyl, Phenyl, Cyan, Hydroxy, Halogenphenyl, Ci-C4-Alkylphenyl oder einen heteroaromatischen Rest substituiertes C₁-C₈-Alkyl,
R² für C₁-C₆-Alkyl, C₂-C₆-Alkenyl, C₂-C₆-Alkinyl, C₃-C₆-Cycloalkyl, Cyclopentenyl, Cyclohexenyl oder durch Halogen, C₁-C₄-Alkoxy oder C₁-C₄-Alkylthio substituiertes C₁-C₆-Alkyl,
R³ und R⁴ unabhängig voneinander für Wasserstoff, Halogen, C₁-C₄-Alkyl, C₁-C₄-Halogenalkyl, C₁-C₄-Alkoxy, C₁-C₄-Alkylthio, C₃-C₆-Cycloalkyl, Cz-C₄-Alkenyl, C₂-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH = CH-CH = CH-, -CH₂-CH=CH-, -(CH₂)₄-, -(CH₂)₃-, -O-CH₂-O-, -O-CH₂-CH₂-O-, -CH₂-0-CH₂-, --(CH₂)₂-CH=CH- oder -CH₂-CH=CH-CH₂-Brücke, welche durch ein bis zwei C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Alkoxy, C₁-C₄-Halogenalkoxy, C₁-C₄-Halogenalkyl, C₁-C₄-Alkylthio, C₁-C₄-Halogenalkylthio, Di-Ci-C₄-alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(C₁-C₂-Alkyl)-, -N(CHO)-, -CH₂-, -CH(CH₃)- oder -C-(CH₃)₂- bedeuten, dadurch gekennzeichnet, dass man ein Anilin der Formel VI 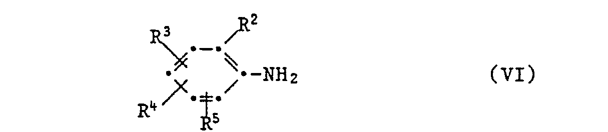 durch Behandlung mit Phosgen in ein Isocyanat der Formel VII 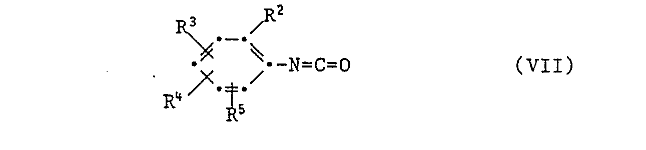 überführt, dieses mit Trimethylazidosilan (N₃-Si(CH₃)₃ oder Triazidoaluminium (Al(N₃)₃) zum 1-phenyl-5-- oxo-2-tetrazolin der Formel VIII 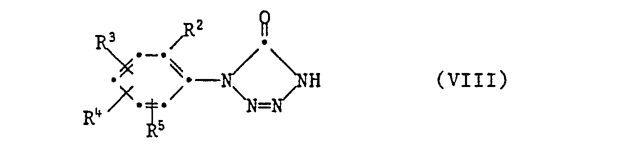 umsetzt, welches man durch Reaktion mit einem Alkylierungsmittel der Formel IV 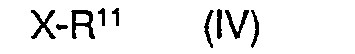 in der 4-Stellung alkyliert, wobei R², R³, R⁴, R⁵ die unter Formel I im Anspruch 1 und R" und X die unter Formel IV mit Anspruch 18 gegebene Bedeutungen haben.

19. Verfahren zur Herstellung von 1-Phenyl-5-oxo-2-tetrazolinen der Formel VIII 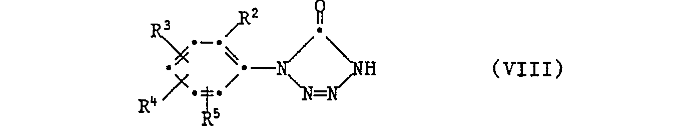 worin
R² für C₁-C₆-Alkyl, C₂-C₆-Alkenyl, C₂-C₆-Alkinyl, Cₛ-C₆-Cycloalkyl, Cyclopentenyl, Cyclohexenyl oder durch Halogen, C₁-C₄-Alkoxy oder C₁-C₄-Alkylthio substituiertes C₁-C₆-Alkyl,
R³ und R⁴ unabhängig voneinander für Wasserstoff, Halogen, C₁-C₄-Alkyl, C₁-C₄-Halogenalkyl, Ci-C₄-Alkoxy, C₁-C₄-Alkylthio, C₃-C₆-Cycloalkyl, C₂-C₄-Alkenyl, C2-C₄-Alkinyl, C₂-C₆-Alkoxyalkyl, C₂-C₆-Alkylthioalkyl, C₁-C₄-Cyanoalkyl, Phenyl-C₂-C₄-alkenyl oder Phenyl-C₂-C₄-alkinyl, oder R³ und R⁴ gemeinsam für eine -CH = CH-CH = CH-, -CH₂-CH = CH-, -(CH₂)₄-, -(CH₂)₃, -O-CH₂-O-, -O-CH₂-CH₂-O-, -CH₂-0-CH₂-, --(CH₂)₂-CH = CH- oder -CH₂-CH = CH-CH₂-Brücke, welche durch ein bis zwie C₁-C₄-Alkylgruppen substituiert sein kann, und
R⁵ für Wasserstoff oder eine Gruppe -Z-R⁶ stehen, wobei
R⁶ Phenyl, Naphthyl, Pyridyl oder durch ein bis zwei Substituenten aus der Gruppe Halogen, C₁-C₄-Alkyl, C₁-C₄-Alkoxy, C₁-C₄-Halogenalkoxy, C₁-C₄-Halogenalkyl, Ci-C₄-Alkylthio, C₁-C₄-Halogenalkylthio, Di-Ci-C₄-alkylamino, Nitro, Cyan, C₁-C₄-Alkoxycarbonyl oder C₁-C₄-Alkylcarbonyl substituiertes Phenyl, Naphthyl oder Pyridyl, und
Z Sauerstoff, Schwefel, eine direkte Bindung, -NH-, -N(Ci-C₂-Alkyl)-, -N(CHO)-, -CH₂-, -CH(CH₃)- oder -C-(CH₃)₂- bedeuten, dadurch gekennzeichnet, dass man ein Anilin der Formel VI 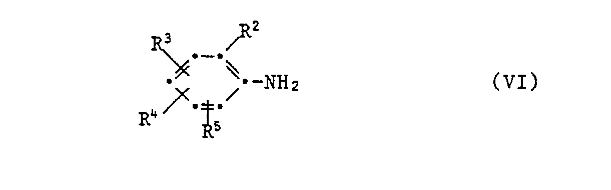 durch Behandlung mit Phosgen in ein Isocyanat der Formel VII 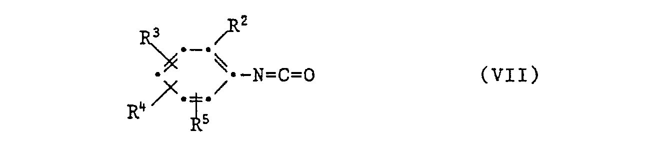
überführt, dieses mit Trimethylazidosilan (N₃-Si(CH₃)₃ oder Triazidoaluminium (Al(N₃)₃) umsetzt, wobei R², R³, R⁴ und R⁵ die unter Formel in Anspruch 1 gegebenen Bedeutungen haben.
